# EUROPEAN PATENT APPLICATION

(11) **EP 4 492 387 A2**
(43) Date of publication of application: **15.01.2025**
(21) Application number: 24215187.6
(22) Date of filing: 19.08.2022
(51) Int. Cl.: G16B 40/30

(54) **COMPUTER-IMPLEMENTED METHODS AND SYSTEMS FOR TRANSCRIPTOMICS**

(30) Priority: 20.08.2021 US 202163235415 P
(62) Divisional of application: 22768668.0
(71) Applicant: Diagenode SA, 4102 Seraing (Ougree) (BE)
(72) Inventor: HITA ARDIACA, Andrea, 4102 Seraing (Ougree) (BE); SCHVARTZMAN, Sol, 4102 Seraing (Ougree) (BE)
(74) Representative: Carpmaels & Ransford LLP

(57) **Abstract**

The present disclosure provides systems and methods for RNA expression quantification comprising hierarchical assignment of RNA sequence reads, communities detection of annotated features and community-level estimation of RNA expression levels.

## Description

### Background

Next Generation Sequencing (NGS) experiments have become the gold standard for many applications within the transcriptomics field, including gene expression profiling, novel transcript discovery, and allele diversity detection. Wang, Zhong et al. "RNA-Seq: a revolutionary tool for transcriptomics." Nature reviews. Genetics vol. 10,1 (2009): 57-63. Kukurba, Kimberly R, and Stephen B Montgomery. "RNA Sequencing and Analysis." Cold Spring Harbor protocols vol. 2015,11 951-69. 13 Apr. 2015.

More sophisticated library preparation methods allow for sequencing and analyzing RNA from individual cells and address further scientific questions such as inferring cell differentiation trajectories, recognizing rare cell populations, and identifying transcription regulatory mechanisms. Hashimshony, Tamar et al. "CEL-Seq: single-cell RNA-Seq by multiplexed linear amplification." Cell reports vol. 2,3 (2012): 666-73. Ziegenhain, Christoph et al. "Comparative Analysis of Single-Cell RNA Sequencing Methods." Molecular cell vol. 65,4 (2017): 631-643.e4. Washietl, Stefan et al. "Computational analysis of noncoding RNAs." Wiley interdisciplinary reviews. RNA vol. 3,6 (2012): 759-78. Hwang, Byungjin et al. "Single-cell RNA sequencing technologies and bioinformatics pipelines." Experimental & molecular medicine vol. 50,8 1-14. 7 Aug. 2018.

While early NGS experiments focused on the detection of polyadenylated RNA (i.e., messenger RNA [mRNA] and polyadenylated long non-coding RNA [lncRNA]), later RNA library preparation methods allow targeting small regulatory RNA (smallRNA), or full transcriptomes (hereafter referred to as total-RNA-seq). Dard-Dascot, Cloelia et al. "Systematic comparison of small RNA library preparation protocols for next-generation sequencing." BMC genomics vol. 19,1 118. 5 Feb. 2018. Yeri, Ashish et al. "Evaluation of commercially available small RNASeq library preparation kits using low input RNA." BMC genomics vol. 19,1 331. 5 May 2018. Roden, C., Mastriano, S., Wang, N., Lu, J.: In: Santulli, G. (ed.) microRNA Expression Profiling: Technologies, Insights, and Prospects, pp. 409-421. Springer, Cham (2015). Total-RNA-seq captures polyadenylated RNA and nonpolyadenylated RNA, which includes all types of smallRNA, lncRNA, and mRNA in both its precursor and processed forms. With total-RNA-seq library preparation methods recently reaching single-cell resolution, a window is opening to investigate transcriptional regulation through non-coding RNA with unprecedented detail. Faridani, Omid R et al. "Single-cell sequencing of the small-RNA transcriptome." Nature biotechnology vol. 34,12 (2016): 1264-1266. Hayashi, Tetsutaro et al. "Single-cell full-length total RNA sequencing uncovers dynamics of recursive splicing and enhancer RNAs." Nature communications vol. 9,1 619. 12 Feb. 2018. Verboom, Karen et al. "SMARTer single cell total RNA sequencing." Nucleic acids research vol. 47,16 (2019): e93. Hagemann-Jensen, Michael et al. "Single-cell RNA counting at allele and isoform resolution using Smart-seq3." Nature biotechnology vol. 38,6 (2020): 708-714. Isakova, Alina et al, "Single cell profiling of total RNA using smart-seq-total." bioRxiv (2020).

Yet, the larger the scope of the RNA biotypes diversity in a dataset, the more challenging it becomes to estimate the relative concentration of transcriptomic content from measured NGS raw reads. Non-coding biotypes are not translated into proteins and vary both in structure and function. Cech, Thomas R, and Joan A Steitz. "The noncoding RNA revolution-trashing old rules to forge new ones." Cell vol. 157,1 (2014): 77-94. Slack, Frank J, and Arul M Chinnaiyan. "The Role of Non-coding RNAs in Oncology." Cell vol. 179,5 (2019): 1033-1055. Statello, Luisa et al. "Gene regulation by long non-coding RNAs and its biological functions." Nature reviews. Molecular cell biology vol. 22,2 (2021): 96-118. This results in highly heterogeneous read profiles that defy standard RNA quantification algorithms.

### Challenges in total-RNA quantification

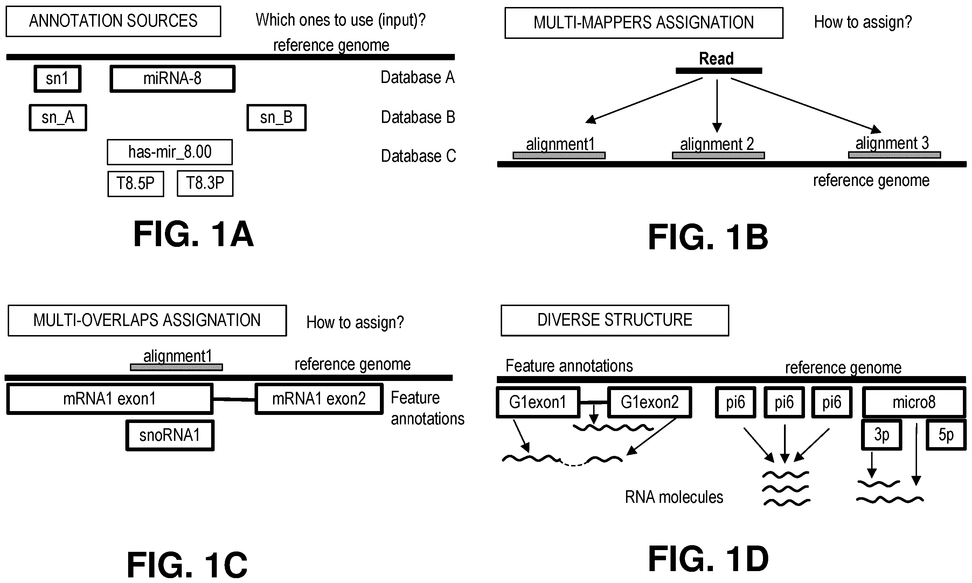

FIGS. 1A-1D illustrate four main challenges that bioinformatic strategies face when quantifying the output of total-RNA-seq experiments. First, the annotations considered constrain the set of RNA features included in the analysis. Standard databases such as Ensembl, Gencode or Refseq store formatted annotations under protein-coding structure (gene, transcript, exon) and exclude less studied small-RNA biotypes (e.g., piRNA). Howe, Kevin L et al. "Ensembl 2021." Nucleic acids research vol. 49,D1 (2021): D884-D891. Frankish, Adam et al. "GENCODE 2021." Nucleic acids research vol. 49,D1 (2021): D916-D923. O'Leary, Nuala A et al. "Reference sequence (RefSeq) database at NCBI: current status, taxonomic expansion, and functional annotation." Nucleic acids research vol. 44,D1 (2016): D733-45. Other biotype-specific databases annotate concrete biotypes in given structure-related formats (e.g., miRbase annotates precursor and mature forms of microRNA together with duplicated loci information). Kozomara, Ana et al. "miRBase: from microRNA sequences to function." Nucleic acids research vol. 47,D1 (2019): D155-D162. Furthermore, less extensively studied RNAs such as piRNAs are annotated in specialized databases only. As a consequence, total-RNA-seq analysis demands integrating multiple databases, homogenizing their formats, and dealing with redundant annotations when these contain slightly different versions of the same transcript (FIG. 1A).

Algorithmically, a major challenge is quantifying reads that align equally well to more than one annotated feature (ambiguous alignments). These reads comprise multi-mappers: reads aligning to multiple genomic locations (FIG. 1B); and multi-overlappers: reads aligning to a genomic location with multiple annotated features (FIG. 1C). This last distinction applies when reads are aligned to a reference genome. In transcriptome alignment, ambiguous assignations typically consist only of multi-mappers rather than multi-mappers and multi-overlappers. While there has been quantification software designed to primarily quantify protein-coding feature expression, this software tends to rely on an assumption that reads can be associated to a unique genomic locus coding for a transcript. This assumption is used to justify the common approach of discarding multi-mapping and multi-overlapping reads, often present in low proportion compared to full transcriptome datasets. But this assumption does not necessarily hold for all genes. Not only can library preparation artifacts cause a read to have an ambiguous origin, but gene recombination, transposition, and duplication events can result in sequence repetition between genes, which explains why sometimes reads cannot be attributed to a single locus unequivocally. Therefore, the discarding approach used by the prior art underrepresents transcripts overlapping with other loci or carrying repeated sequences from other loci. In addition, smallRNAs pose a more extreme paradigm since their short transcript lengths combined with post-transcriptional modifications and multiple loci coding for the same transcript result in more abundant multi-mappers, while their genomic origin embedded within a longer RNA exon or intron results in more abundant multi-overlaps. Boivin, Vincent et al. "Protein coding genes as hosts for noncoding RNA expression." Seminars in cell & developmental biology vol. 75 (2018): 3-12. In this context, dealing with ambiguous alignments becomes a requirement to quantify many transcripts. Furthermore, distinct smallRNA classes, such as microRNA (miRNA), piRNA, snRNA, rRNA, scaRNA, snoRNA, tRNA, tRF, YRNA and vaultRNA, differ in nature, preventing a common structural characterization.

Different strategies have been proposed to quantify multi-mappers and multi-overlappers. Raw counting tools quantify all multi-mappers and multi-overlappers through decision rules such as randomly selecting one alignment or fractionally counting all alignments. Anders, Simon et al. "HTSeq--a Python framework to work with high-throughput sequencing data." Bioinformatics (Oxford, England) vol. 31,2 (2015): 166-9. Liao, Yang et al., featureCounts: an efficient general purpose program for assigning sequence reads to genomic features, Bioinformatics, Volume 30, Issue 7, 1 April 2014, Pages 923-930. These split ambiguity over all loci candidates uniformly, ignoring any other pattern foretelling its active/inactive condition. More refined tools address ambiguity, but only in restricted biotypes and contexts: Focused on longRNA, probabilistic approaches statistically weight transcript candidates, including RSEM, which resolves ambiguity from sequence repetition in mRNA isoforms with weighted counts based on a Bayesian model and pseudoaligners such as Kallisto and Salmon, and are more suitable for quantifying well-characterized transcriptome as reads are aligned only to transcript sequences. Li, Bo, and Colin N Dewey. "RSEM: accurate transcript quantification from RNA-Seq data with or without a reference genome." BMC bioinformatics vol. 12 323. 4 Aug. 2011. Bray, Nicolas L et al. "Near-optimal probabilistic RNA-seq quantification." Nature biotechnology vol. 34,5 (2016): 525-7. Patro, Rob et al. "Salmon provides fast and bias-aware quantification of transcript expression." Nature methods vol. 14,4 (2017): 417-419. Oriented to smallRNA data, a few algorithms consider neighboring patterns around each multi-mapping alignment (e.g., in terms of uniquely-mapping alignments coverage and presence of annotations) in order to estimate a distinct weight for each smallRNA multi-mapped. Johnson, Nathan R et al. "Improved Placement of Multi-mapping Small RNAs." G3 (Bethesda, Md.) vol. 6,7 2103-11. 7 Jul. 2016. Handzlik, Joanna E et al. "Manatee: detection and quantification of small non-coding RNAs from next-generation sequencing data." Scientific reports vol. 10,1 705. 20 Jan. 2020. Also, rescue methods prioritize features with more uniquely-mapping alignments, assuming that these agglomerate in active loci and multi-mappers result from partial-sequence repetition with inactive loci pseudo copies. Deschamps-Francoeur, Gabrielle et al. "CoCo: RNA-seq read assignment correction for nested genes and multimapped reads." Bioinformatics (Oxford, England) vol. 35,23 (2019): 5039-5047. Kaminow, Benjamin et al. "Starsolo: accurate, fast and versatile mapping/quantification of single-cell and single-nucleus RNA-seq data." bioRxiv (2021). Zytnicki (2017) proposes to report multi-mappers as merged gene counts, which may result in some annotated features quantified in multiple merged output feature. Zytnicki, Matthias. "mmquant: how to count multi-mapping reads?." BMC bioinformatics vol. 18,1 411. 15 Sep. 2017. GeneQC employs a Machine Learning approach to provide with uncertainty estimates for each feature related to its alignments' ambiguity. McDermaid, Adam et al. "A New Machine Learning-Based Framework for Mapping Uncertainty Analysis in RNA-Seq Read Alignment and Gene Expression Estimation." Frontiers in genetics vol. 9 313. 14 Aug. 2018. Deschamps-Francoeur et al., (2020) review multi-mapping handling strategies and their current limitations. Deschamps-Francoeur, Gabrielle et al. "Handling multi-mapped reads in RNA-seq." Computational and structural biotechnology journal vol. 18 1569-1576. 12 Jun. 2020. In summary, there remains a need for systems and methods capable of resolving ambiguities from multi-mappers and multi-overlappers that can be applied to the whole gamut of RNA biotypes.

Finally, biotype-specific pipelines quantify expression levels by taking into account the genomic structure of the biotype in question. This raises a conceptual question: what is the suitable feature output-level at which transcript abundance estimation is more meaningful for total-RNA-seq? FIG. 1D illustrates that total-RNA-seq integrates heterogeneous relations between transcript-loci and transcript-product: a cluster of smallRNA loci (e.g. pi6, from the piRNA biotype) can be actively transcribing the same product; microRNA transcripts result in two distinct processed mature transcripts (e.g. unprocessed mi8 is post-clipped to 3p/5p mature forms); long transcripts comprising exons and introns undergo splicing resulting in both reads from the precursor and mature transcript forms. This heterogeneity challenges determining an equally suitable output-level for the quantification of all RNA molecules simultaneously.

Biotype-specific pipelines may quantify expression levels depending on the biotype structure, according to the state-of-the-art research and the feature annotations information available. On one hand, protein-coding feature abundance has conventionally been summarized at isoform-level or gene-level (collapsing corresponding exons). In addition, unspliced and spliced reads can be distinctly quantified (e.g., to estimate the RNA velocity of single cells). La Manno, Gioele et al. "RNA velocity of single cells." Nature vol. 560,7719 (2018): 494-498. On the other hand, smallRNA tools quantify at transcript level, integrating annotations from specific non-coding databases and reporting biotype-specific outputs if annotated information allows, e.g., given repeated loci of a same smallRNA, counts are sumup at RNA product level; post-processed smallRNA can be distinctly quantified (mature microRNA, tRNA derived fragments, etc.). An, Jiyuan et al. "miRDeep*: an integrated application tool for miRNA identification from RNA sequencing data." Nucleic acids research vol. 41,2 (2013): 727-37. Stocks, Matthew B et al. "The UEA sRNA Workbench (version 4.4): a comprehensive suite of tools for analyzing miRNAs and sRNAs." Bioinformatics (Oxford, England) vol. 34,19 (2018): 3382-3384. Kuksa, Pavel P et al. "SPAR: small RNA-seq portal for analysis of sequencing experiments." Nucleic acids research vol. 46,W1 (2018): W36-W42. Liu, Qi et al. "Small noncoding RNA discovery and profiling with sRNAtools based on high-throughput sequencing." Briefings in bioinformatics vol. 22,1 (2021): 463-473. Fehlmann, Tobias et al. "miRMaster 2.0: multi-species non-coding RNA sequencing analyses at scale." Nucleic acids research vol. 49,W1 (2021): W397-W408. In this regard, total-RNA-seq analysis demands a flexible approach that adaptively defines feature quantification output-levels suiting all RNA biotypes, independently of the available annotations.

Taken together, improvements are needed to systems and methods that perform simultaneous quantification of smallRNA, long intronic or non-coding RNA (e.g., lncRNA), and long exonic RNA (e.g., mRNA). Improving these systems and methods requires new strategies that account for the diverse nature of each transcript, without relying on assumptions that could lead to biotype-dependent quantification biases.

### Brief Summary of the Disclosure

The present disclosure addresses the unmet needs of the prior art, as described above. In some embodiments, the present disclosure provides a global framework to resolve the prevalent multi-overlapping and multi-mapping issues that arise in transcriptomics. The present disclosure facilitates unambiguous quantification of RNA expression from RNA-seq data (e.g., RNA sequence reads). The systems and methods of the present disclosure further provide an output compatible with downstream processing or analysis of RNA-seq data for investigatory and/or clinical purposes.

The present disclosure provides novel RNA-seq quantification systems and methods that hierarchically assigns reads to smallRNA and longRNA, accounting for their transcript length disparity to resolve multi-overlapping events. The systems and methods of the present disclosure model multi-mapping alignments in a biotype-specific graph that is exploited to detect and report expression of sequence-similar annotated loci as integrated features. The systems and methods described herein are generalizable to the simultaneous quantification of multiple RNA biotypes, compatible with any genome and annotations set, and compatible with single-cell data. In at least one embodiment, the systems and methods described herein can be executed as a single command-line program.

Further, and among other advantages, the systems and methods disclosed herein resolve multi-overlapping situations that arise in transcriptomics due to smallRNA originating within longRNA exons or introns; take into account both polyadenylated and nonpolyadenylated reads from longRNA; assign multi-mapping reads with heterogeneous profiles (which abound in smallRNA); and provide an output expression level in an adaptive data-driven manner.

In some embodiments, the present disclosure provides a computer-implemented method for quantification of RNA expression from RNA sequence reads, comprising: (a) hierarchically assigning RNA sequence reads from at least one cell to RNA feature annotations according to RNA transcript length, to yield feature-read pairs assigned as small RNA, long exonic RNA, or long intronic RNA, and (b) detecting communities of highlyrelated features of the feature-read pairs thereby quantifying expression of RNA sequence reads, including communities of RNA sequence reads. Thus, in some embodiments, the method comprises quantifying transcriptomic expression at an adaptive output-level, wherein the output comprises aggregated individual multi-mapped features that have been quantified as a community. In some embodiments, the method comprises outputting quantification of RNA expression in an RNA expression matrix. In some embodiments, the method comprises assigning a biotype to a plurality of the detected communities. In some embodiments, the computer-implemented method further comprises, prior to (a), the RNA sequence reads are collected or have been collected from a library of detected RNA sequences (RNA-seq). In some embodiments, the method further comprises, prior to (a), trimming the collected RNA sequence reads to remove artificial sequences.

In some embodiments, the hierarchical assignation of the method comprises assigning RNA sequence reads that map to overlapping coordinates of the genome. In some embodiments, the hierarchical assignation comprises assigning RNA sequence reads overlapping a smallRNA feature and a longRNA feature as smallRNA, followed by assigning RNA sequence reads as long exonic RNA, followed by assigning remaining RNA sequence reads as long intronic RNA.

In some embodiments, prior to (a) of the method, the RNA sequence reads are aligned or have been aligned to a reference genome.

In some embodiments, the method comprises detecting communities from a graph of feature-read pairs, wherein each vertex of the graph is a transcriptomic feature and edges connect two vertices for which multi-mapping reads exist. In some embodiments, the edges between two vertices comprise pairs of edges. In some embodiments, each vertex is weighted to have a size proportional to its number of aligned reads, and detecting communities accounts for vertex weights. In some embodiments, each edge is weighted to have a thickness proportional to the fraction of multi-mappers shared by the vertices the edge connects, over total aligned reads, and detecting communities accounts for edge weights.

In some embodiments, detecting communities comprises performing the Rosvall Map Equation to detect highly related communities.

In some embodiments, the RNA sequence reads from at least one cell are from a single cell. In some embodiments, the RNA sequence reads from at least one cell are from multiple cells.

In some embodiments, the present disclosure provides a computer-readable medium for performing a computer-implemented method as described herein.

In some embodiments, the present disclosure provides a system, comprising a processor and instructions configured to cause the processor to perform a computer-implemented method as described herein.

### Brief Description of the Drawings

**FIGs. 1A-1D** illustrate four main challenges in total-RNA-seq quantification. FIG. **1A** illustrates that integration of databases from multiple sources can lead to redundant annotations of the same feature with different names, formats, or slightly different coordinates. For example, FIG. 1A illustrates a scenario in which the sources are Database A (including sn1 and miRNA-8), Database B (including sn_A and sn_B), and Database C (including has-mir_8.00, T8.5P, and T8.3P). FIG. 1B provides an example of a multimapping read (black line) that aligns equally well to more than 1 position (gray lines) and cannot be assigned to a genomic origin unequivocally. FIG. 1C provides an example of a multi-overlapping read that maps to a genomic position where two annotated features coexist (an mRNA exon and a snoRNA) and cannot be assigned to a feature unequivocally. **FIG. 1D** illustrates that total-RNA-seq integrates heterogeneous relations between transcript-loci and transcript-product and that this heterogeneity challenges determining an equally suitable output-level for the quantification of all RNA molecules simultaneously.
**FIG. 2A** illustrates an embodiment of hierarchical assignation of the present disclosure. Alignments are overlapped with feature annotations in 3 sequential rounds: smallRNA (dashed line), longRNA exons (solid line), and longRNA introns (dotted line).
**FIG. 2B** illustrates an embodiment of communities detection of the present disclosure.
**FIG. 2C** illustrates an exemplary embodiment of the present disclosure encompassing RNA-seq data pre-processing and "MGcount."

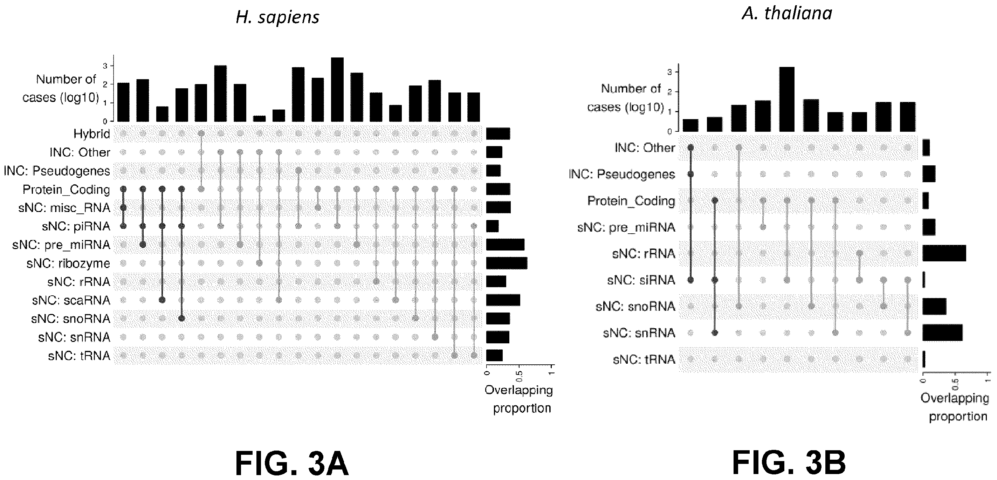

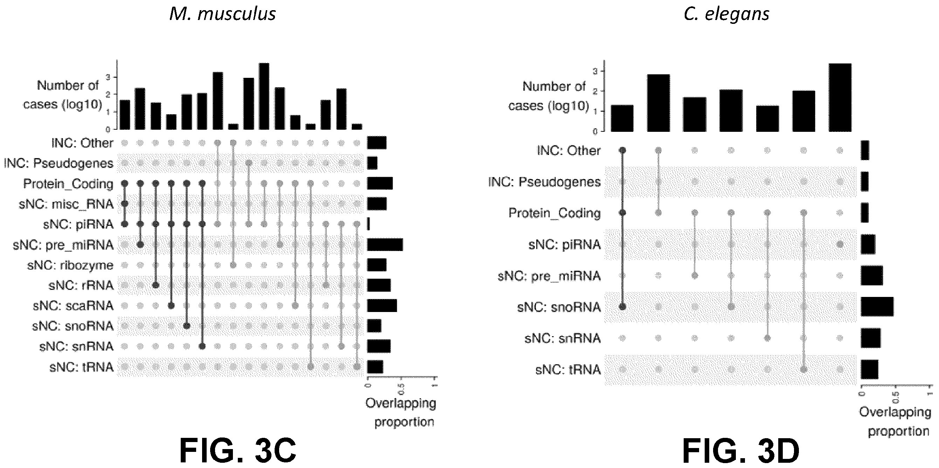

**FIGs. 3A-3D** illustrate frequencies of annotated transcriptomic features overlapping in genomic origin by biotype and organism. In each panel, the dotplot presents combinations of two (medium gray) or three (dark gray) overlapping features of different biotypes whose occurrence exceeds the 5% of the total number of features in the genome from the less abundant biotype in the combination. In each panel, the top barplot shows the log 10 of the total number of cases per combination. The right barplot shows the relative proportion of features overlapping with any other feature by biotype (lNC: long non-coding; sNC: small non-coding).
**FIGs. 4A-4B** illustrate distributions of counts obtained under different multi-overlapping assignation rules in the presence and absence of smallRNA-longRNA overlap for all smallRNA and longRNA features. Stars correspond to the p-value level of significance of a TOST equivalence test with equivalence margins at one standard deviation of the reference distribution.
**FIG. 4C** provides pairwise comparison matrices displaying normalized mutual information (NMI) between communities solutions generated by MGcount, an exemplary embodiment of the present disclosure.
**FIG. 4D** shows a linear regression fit (dashed black line) and its 95% confidence level interval (gray area) modeling the relationship between RT-qPCR and NGS fold change estimation of Human Brain and K562 expression for 20 smallRNA markers of different biotypes.
**FIGs. 5A-5C** and **6A-6F** present visualizations of the graphs obtained from the pool of human libraries (K562 cell line and Human Brain) in which vertices are annotated features from the gtf with size proportional to their respective number of aligned reads; edges represent shared multi-mapping reads between the vertices, each edge connecting two features with shared multi-mappers with thickness proportional to the fraction of shared multi-mappers over the total alignments; and gray areas delineate the MG communities detected by MGcount, an exemplary embodiment of the present disclosure.
**FIGs. 7A-7D** illustrate RNA expression quantification with different counting approaches. (sNC: small non-coding; lNC: long non-coding).

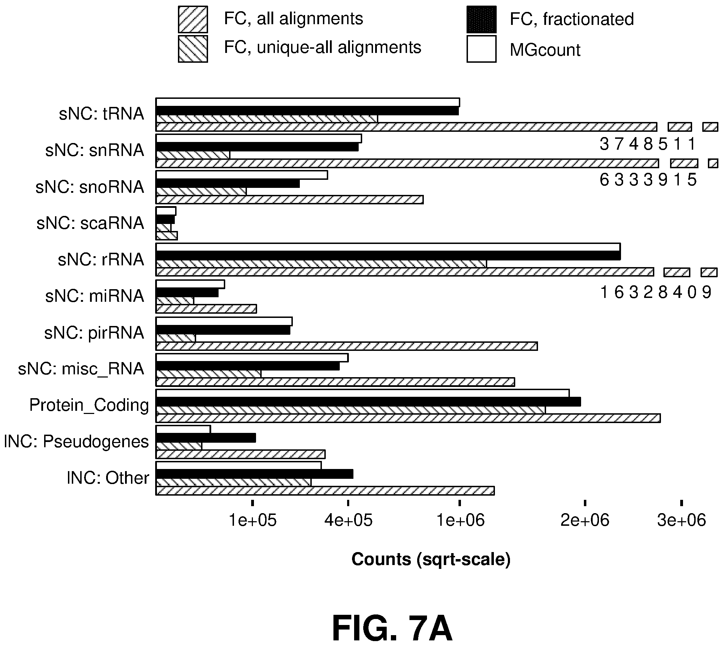

**FIG. 7A** compares counts when alignments are clustered and weighted according to MGcount, an exemplary embodiment of the present disclosure (top bar); when all alignments are fractionally counted as 1 divided by the number of multi-assignments (second bar from top), when only uniquely-mapping reads are counted (second bar from bottom), and when all alignments are counted (bottom bar).

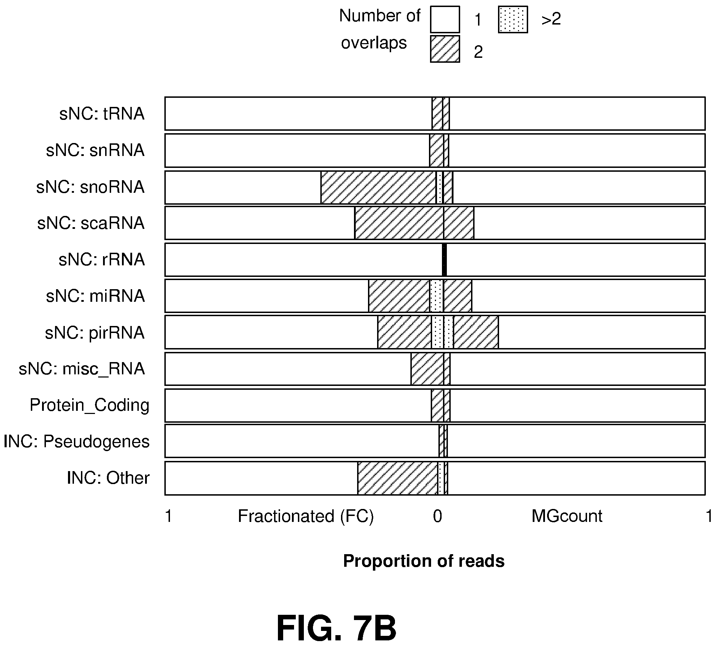

**FIG. 7B** compares the proportion of reads overlapping to 1, 2 or more annotations according to raw alignments assignation (left) and after an exemplary embodiment of the present disclosure (MGcount) assigns by hierarchical rounds (right). The order of bars, from outermost to centermost, is: reads with 1 annotation, reads with 2 annotation overlaps, reads with more than 2 annotation overlaps.

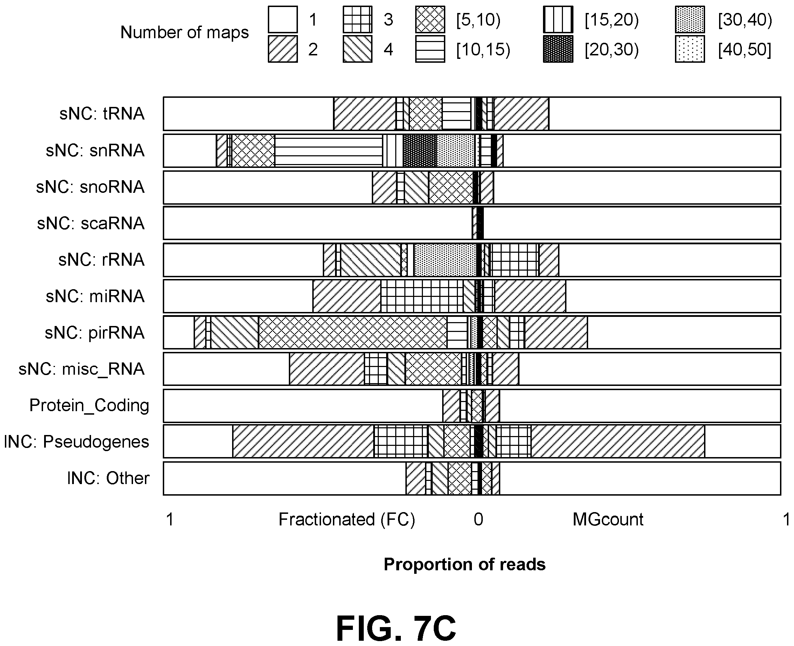

**FIG. 7C** compares the proportion of reads multi-mapping to a given number of genomic locations (up to 50), according to raw alignments assignation (left) and after an exemplary embodiment of the present disclosure (MGcount) collapses multi-mappers to features in the same community (right). The order of bars, from outermost to centermost, is: reads mapping to 1 location, reads mapping to 2 locations, reads mapping to 3 locations, reads mapping to 4 locations, reads mapping to 5-9 locations, reads mapping to 10-14 locations, reads mapping to 15-19 locations, reads mapping to 20-29 locations, reads mapping to 30-39 locations, reads mapping to 40-50 locations.

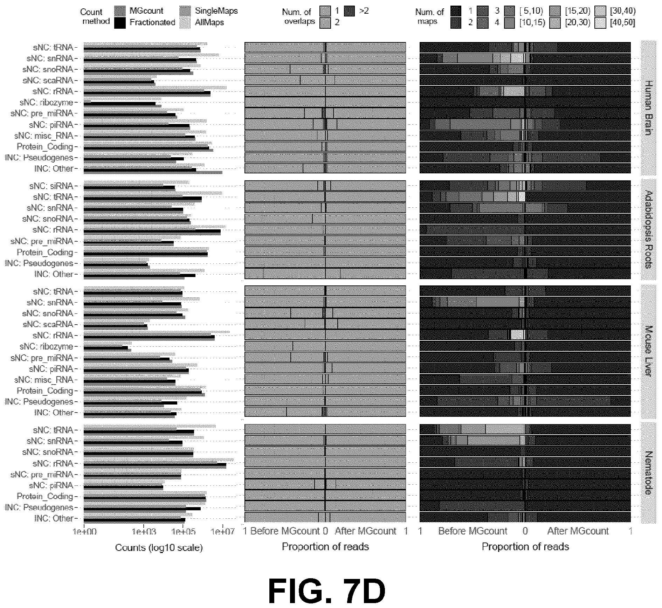

**FIG. 7D** represents the data of FIGs.. 7A-7C, broken down by organism of origin (human brain, *Arabidopsis* roots, mouse liver, and nematode). The order of bars in the Counts (log10 scale) data column is: counts using method of counting all alignments (top), counts using method of counting only uniquely-mapping reads (second from top), counts using method of fractionally counting all alignments as 1 divided by the number of multi-assignments (second bar from bottom), counts using method of counting alignments clustered and weighted according to MGcount, an exemplary embodiment of the present disclosure (bottom bar). The order of bars in the Proportion of Reads (multi-overlap) data column, from outermost to centermost, is: reads with 1 annotation, reads with 2 annotation overlaps, reads with more than 2 annotation overlaps. The order of bars in the Proportion of Reads (multi-map) data column, from outermost to centermost, is: reads mapping to 1 location, reads mapping to 2 locations, reads mapping to 3 locations, reads mapping to 4 locations, reads mapping to 5-9 locations, reads mapping to 10-14 locations, reads mapping to 15-19 locations, reads mapping to 20-29 locations, reads mapping to 30-39 locations, reads mapping to 40-50 locations.

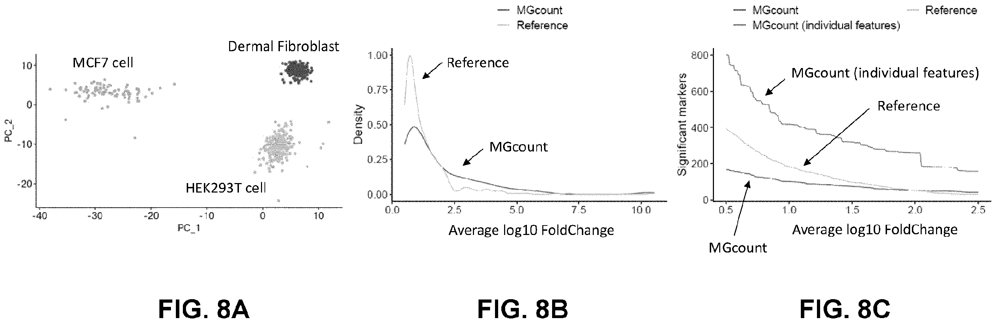

**FIGs. 8A-8C** provide further quantification outputs in a single-cell case example dataset, as performed using MGcount, an exemplary embodiment of the present disclosure. **FIG. 8A** illustrates sample representation in the Principal Component Analysis (PCA) graphical space defined by the first and second components. Each point is a cell, colored by cell line of origin. (PC: Principal Component). **FIG. 8B** provides density distributions of the logarithmic (log10) fold change of all differentially expressed markers between cell populations with a p-value below 0.05 and a natural log fold change above 0.5. **FIG. 8C** provides the number of statistically significant features (p-value below 0.05) detected by average of the log10 fold change.

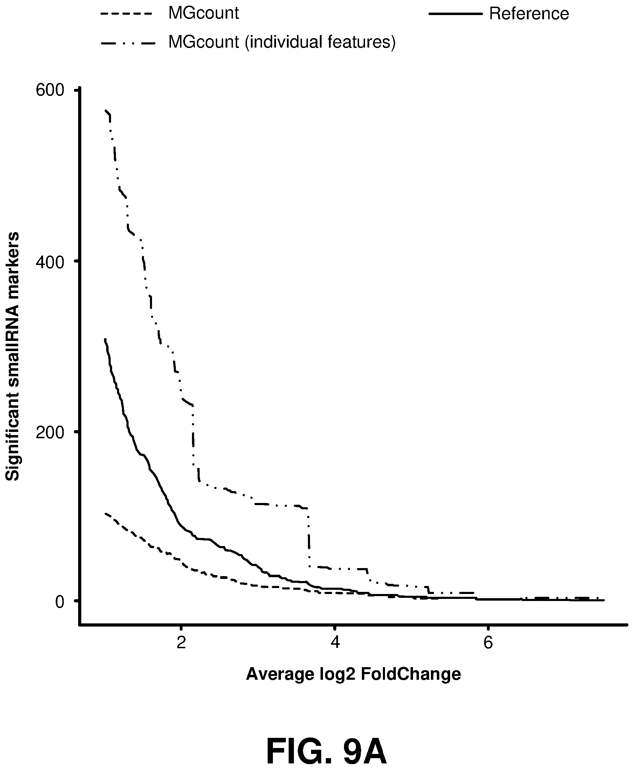

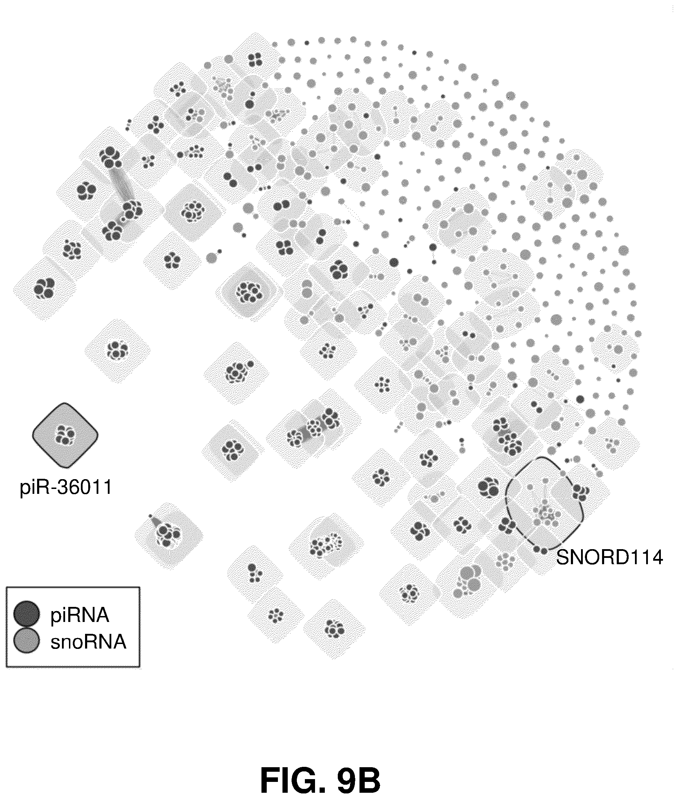

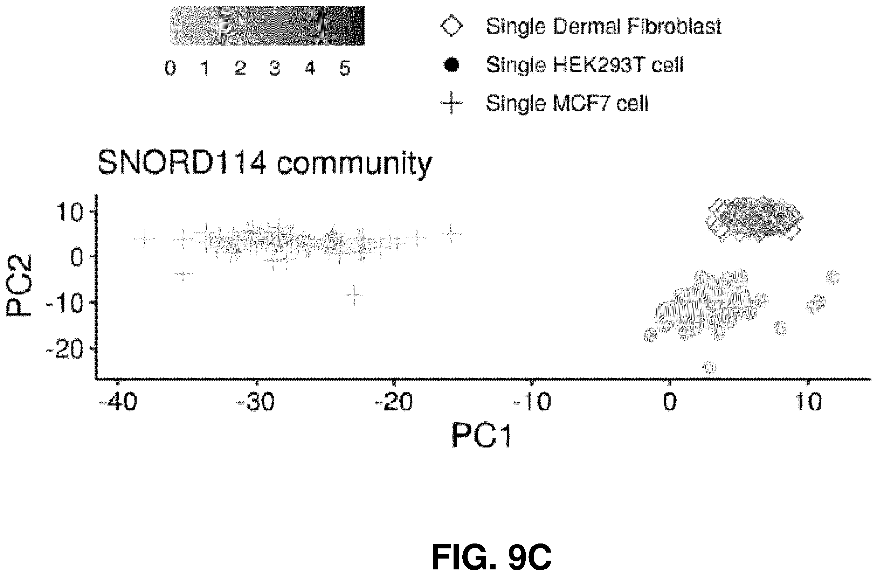

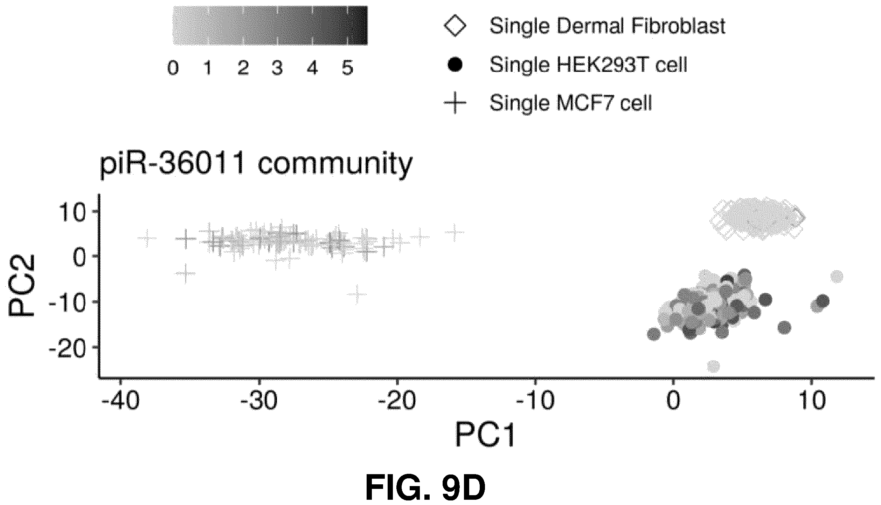

**FIGs. 9A-9D** provide quantification outputs in a single-cell case example dataset, as performed using MGcount, an exemplary embodiment of the present disclosure. **FIG. 9A** shows the number of statistically significant features (p-value below 0.05) detected by log2 fold-change average. **FIG. 9B** is an MGcount sub-graph for snoRNAs and piRNAs where snord-114 and piR-36011 communities are highlighted. **FIG. 9C** shows the expression level of the snord-114 community by a cell represented in the PC1-PC2 space. **FIG. 9D** shows the expression level of the piR-36011 community by a cell represented in the PC1-PC2 space. (PC: Principal Component).

### Detailed Description of the Disclosure

The embodiments of the present disclosure include systems and methods that comprise read-to-feature assignation followed by communities detection.

In some embodiments, the method may further comprise collecting RNA-seq data. In some embodiments, the method may further comprise trimming the RNA sequence reads to remove artificial sequences. In some embodiments, the RNA sequence reads are aligned to a reference genome. In some embodiments, alignment comprises deduplication.

Inputs to the read-to-feature assignation element of the method comprise genome alignments of RNA-seq reads and a transcriptomic features annotation file. An input may be RNA sequence reads or may be RNA sequence reads that have been aligned to a reference genome. The reads (optionally prior to alignment) may be trimmed to remove artificial sequences. Unless specified otherwise, "read" (in the context of RNA sequence reads) refers to RNA sequence reads and RNA sequence/genome alignments, which may or may not have been trimmed or deduplicated.

In some embodiments, the input features annotation file may be a .gtf (Gene Transfer Format) file. In some embodiments, the features annotation file may comprise more than one .gtf file. Feature annotation files may be sources from databases known in the art. Exemplary sources for feature annotation files include, but art not limited to DASHR, RNAcentral, miRbase, Ensembl, or a combination thereof. In some embodiments, the feature annotation file or files used in the method compile annotations from each of DASHR, RNAcentral, miRbase and Ensembl. The feature annotation file may be species-specific or may account for annotations from multiple species. In some embodiments, the feature annotation file is specific to any species. In some embodiments, the feature annotation file comprises annotations specific to the human genome. In some embodiments, the feature annotation file comprises annotations specific to the *Arabidopsis* genome. In some embodiments, the feature annotation file comprises annotations specific to the mouse genome. In some embodiments, the feature annotation file comprises annotations specific to the nematode genome.

Read-to-feature assignation may comprise assigning genome (RNA) alignments or reads to RNA feature annotations, including in the event of overlap in genomic coordinates. The read-to-feature assignation comprises hierarchical assignation based on RNA transcript length. For example, the method may assign RNA-seq reads to RNA feature annotations in three sequential rounds. In certain embodiments, the sequential rounds have a hierarchy of: smallRNA, then long exonic RNA (longRNA exon), then long intronic RNA (longRNA intron). Without limitation, smallRNA may comprise microRNA (miRNA), piRNA, snRNA, rRNA, tRNA, scaRNA, snoRNA, tRF, YRNA, and vaultRNA. As a non-limiting example, a read with coordinates overlapping both a smallRNA feature and a longRNA feature would be assigned as a smallRNA. Reads with at least one alignment overlapping with an annotation in the current round are assigned to such feature and skipped in subsequent rounds.

In some embodiments, alignment-to-feature assignation accounts for at least feature type, feature coordinates, and a minimum overlapping fraction of the alignment required to assign it to the annotated feature. In some embodiments, feature biotype determines a read's membership to a round (e.g., smallRNA, longRNA exon, longRNA intron). At the alignment-to-feature assignation stage, different alignments of a read may be assigned to different features.

Outputs of the alignment-to-feature assignation element of the method comprise feature-alignment pairs assigned according to transcript length and assignation rounds (e.g., assigned as smallRNA, longRNA exon, or longRNA intron).

At each assignation round, alignment-to-feature overlapping may be implemented using a counting software known in the art.

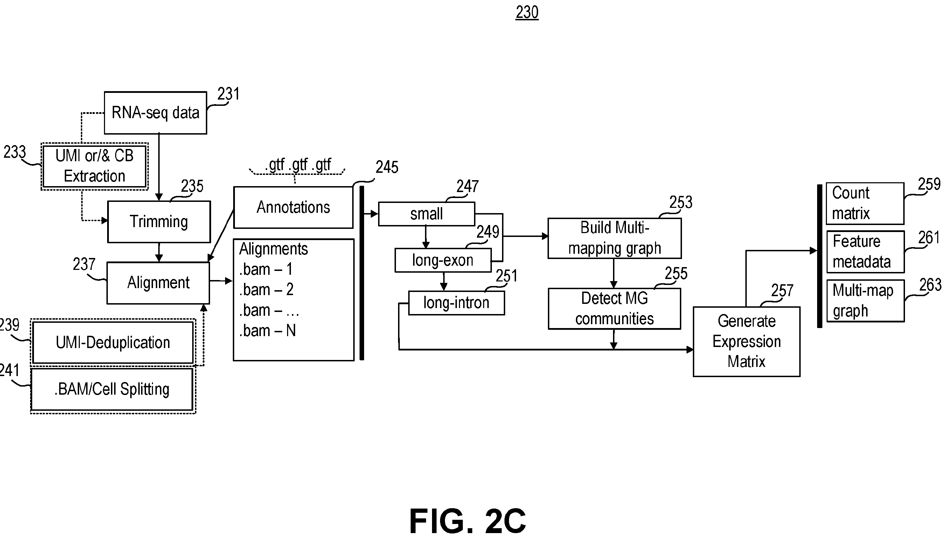

Disambiguation of reads and features (read-to-feature assignation) from alignment-to-feature assignation occurs with communities detection. In some embodiments, read-to-feature assignation comprises use of known algorithms, such as FeatureCounts, discussed below with respect to FIG. 2C. In some embodiments, read-to-feature assignation uses other known algorithms such as HTSeq-count. One of ordinary skill will understand that other algorithms for read-to-feature assignation are possible (including, e.g., brute force comparison).

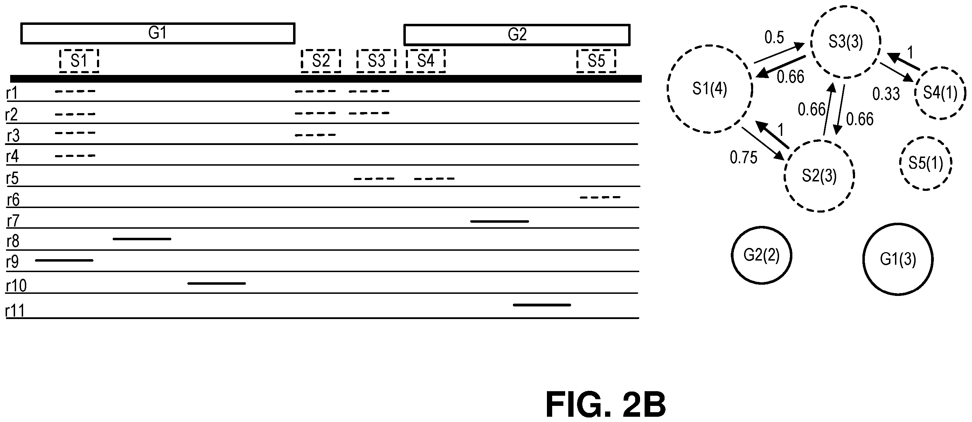

An input to the communities detection element of the method may comprise a graph. In some embodiments, read-to-feature multi-mapping graphs are separately constructed for smallRNA and exonic longRNA rounds assignments. In some embodiments, a multi-mapping graph (e.g., FIG. 2B) is built such that each vertex is a feature and edges connect vertices with common multi-mapping reads. In some embodiments, the edges comprise pairs of edges. In some embodiments, the pairs of edges have weights proportional to the number of multi-mappers. In certain embodiments, edge weights are defined as the ratio of multi-mapping reads between the two vertices normalized by the total number of reads assigned to the source vertex. In some embodiments, vertex weights are defined as the log-transformed number of assigned alignments. In some embodiments, the method assigns non-uniform teleporting probabilities proportionally to the vertices' weight, correcting for over visit frequencies to features with fewer alignments. In some embodiments, the method ignores weak edges for community detection. In some such embodiments, the thresholds for weak edges are common to all biotypes. In some embodiments, the thresholds for weak edges are determined by the user. In some embodiments, the high threshold is 0.75 and/or the low threshold is 0.01. In some embodiments, edges below the high threshold are ignored for a longRNA graph. In some embodiments, the threshold closer to the graph weights' first quantile is employed for each biotype to prevent over-fitting the smallRNA graph (splitting of large densely connected communities and merging of small loosely connected communities) according to the weights' distribution of a graph.

Communities detection comprises unsupervised clustering, e.g., of densely connected vertices in the graph. In some embodiments, the communities detection element of the method accounts for graph directionality (e.g., weights proportional to the number of multi-mappers). In some embodiments, the communities detection element of the method does not account for graph directionality. In some embodiments, the communities detection element of the method accounts for a modularity score. In some embodiments, the communities detection element of the method does not account for a modularity score. In some embodiment, the communities detection element of the method formulates the description length of an infinite random walk trajectory along the graph as a function of its topology, so as to minimize the description length by identifying the communities for which the random walk stays the maximum within and moves the minimum between.

FIG. 2B illustrates an example of multi-mapping graph generation in which reads *rᵢ* (*i* = 1, 11) have been assigned to smallRNA annotations *S₁*, *S₂*, *S₃*, *S₄*, *S₅* and longRNA *G₁*, *G₂* annotations sequentially. As indicated by FIG. 2B, *r₁* can be mapped to *S₁, G₁*, *S₂* and *S₃* but because of the hierarchy, it will be directly assigned to *S₁*, *S₂* and *S₃* simultaneously increasing by one unit the edges weight between the 3; *r₅* is a uniquely mapping read overlapping *G₂* and *S₅* but it becomes *S₅* for the hierarchy; *r₁₀* becomes *G₂* because it falls outside *S₅.* For the uniquely-mappers, no edges are created. At the end, all edges' weights are normalized by the total number of alignments of the source vertex. Hence, the weight of the edge connecting *S₁* with *S₂* weight becomes 3/4 (reads mapping both *S₁* and *S₂* divided by reads aligned to *S₁*).

In some embodiments, the communities detection element of the method is implemented using a graph clustering algorithm. In some embodiments, the communities detection element of the method is implemented using the Rosvall Map Equation (Rosvall et al., 2008). Alternative graph clustering approaches may include "Walktrap," "Spinglass," "Label propagation," Louvain," or "Greedy optimization."

Outputs of the communities detection element of the method comprise communities of vertices. Outputs of the communities detection element of the method may further comprise isolated vertices.

In some embodiments, the methods or software described herein generate an output expression matrix for each hierarchical assignation round, appended together in a single output matrix. In such embodiments, for each read, each alignment first gets a 1/N count, where N is the number of multi-mappers or residual multi-overlaps within biotypes that survive the hierarchical assignment. Next, counts for annotations which have been aggregated together in a community by the map equation are summed up. In this way, the systematic ambiguity in multi-mapping reads gets collapsed into a single community while the remaining signal is reported as fractionated counts.

In some embodiments, the methods or software further generate an output of features metadata. For example, the metadata may comprise information on whether a feature is a unique feature of a community features, as well as its biotype. In some embodiments, the methods or software further generate an output of a multi-mapping graph adjacency matrix, which comprises links between annotated features. The adjacency matrix may permit reconstructing and/or analyzing a smallRNA multi-mapping graph and/or longRNA multimapping graph. In some embodiments, the methods or software further generate an output of multi-mapping graph communities. The multi-mapping graph communities may comprise a table linking each original feature in the gtf input file(s) with the resultant feature matching count matrix and feature metadata identifiers. In some embodiments, the multi-mapping graph communities comprises aggregated features and unique features. In some embodiments, the multi-mapping graph communities table comprises a total number of alignments per feature.

A non-limiting but exemplary embodiment of the present disclosure is the RNA-seq quantification system Multi-Graph count ("MGcount"). A schematic of a method of the disclosure as implemented using MGcount is provided in FIG. 2C. As shown in FIG. 2C, MGcount takes a set of genomic alignments (.bam or Binary sequence alignment/map (SAM) format files) and an RNA feature annotations file (.gtf) as inputs. The algorithm assigns reads hierarchically and then models multi-mapping reads assignment in a graph using the Rosvall "map equation" methodology. As output, MGcount provides an RNA expression count matrix (where feature clusters are collapsed as new defined features), a feature metadata table and the generated multi-mapping graphs.

### Exemplary Embodiments: MGcount

Exemplary embodiments of RNA-seq data pre-processing and MGcount are discussed below with respect to FIG. 2C. One of ordinary skill in the art would understand that, in some embodiments, the operations in FIG. 2C (as well as other embodiments in this disclosure) may be executed on a suitable computing device.

Suitable computing devices may include, for example, a personal computer, a desktop computer, a laptop computer, a tablet computer, a smartphone, a server, or the like. Suitable computing devices may include, for example, one or more processors may include one or more known processing devices, such as a microprocessor manufactured by Intel^{™}, AMD^{™}, Samsung^{™}, Qualcomm^{™}, or others. The one or more processors may constitute a single core or multiple core processors that can execute parallel processes simultaneously, may be configured with virtual processing technologies, may be configured with logical processors to simultaneously execute and control multiple processes, may implement virtual machine technologies, or other known technologies to provide the ability to execute, control, run, manipulate, store, etc., multiple software processes, applications, programs, etc. In other embodiments, the one or more processors may include a multiple-core processor arrangement (e.g., dual, quad core, etc.) configured to provide parallel processing functionalities.

Suitable computing devices may include, for example, one or more storage devices. The one or more storage devices, in some embodiments, may be one or more of volatile, nonvolatile, tape, optical, semiconductor, magnetic, non-removable, removable, or other type of storage device or tangible computer-readable medium that stores data. Data may include, for example, data associated with computer program applications (including, e.g., applications implementing the disclosed embodiments), data used by computer program applications, or other applications, including operating systems that perform known operating system functions when executed by one or more processors.

Suitable computing devices may include other devices, including, for example, network devices (e.g., devices configured to communicate data to other computing systems), display devices (e.g., devices configured to communicate data to a display for presentation to a human user), input devices (e.g., devices configured to receive signals), output devices (e.g., devices configured to emit signals), or the like.

FIG. 2C illustrates an overview of RNA-seq data pre-processing and "MGCount," an exemplary embodiment of the present disclosure. In particular, FIG. 2C illustrates a method 230. In some embodiments, method 230 may be executed by one or more processors of one or more suitable computing devices, as discussed above. As one of ordinary skill will appreciate, each step in FIG. 2C may be duplicated, omitted, performed in a different order, or modified in accordance with the understanding of one of ordinary skill.

Method 230 starts at step 231. In step 231, one or more processors may receive RNA-seq reads. In some embodiments, these RNA-seq reads may be received in the known fastq file formats; in other embodiments, these RNA-seq reads may be received in other formats. In some embodiments, one fastq file may be received per sample or per cell. In some embodiments, the one or more processors may automatically detect the read input format, thus not requiring the user to specify it.

In step 233, the one or more processors may extract UMIs (unique molecular identifiers) and/or CBs (cell barcodes). In some embodiments, extraction in step 233 may be performed using Cell Ranger, UMI-tools, scPipe, fastp, zUMIs, kallisto, salmon, or alevin. Zheng, Grace X Y et al. "Massively parallel digital transcriptional profiling of single cells." Nature communications vol. 8 14049. 16 Jan. 2017. Smith, Tom et al. "UMI-tools: modeling sequencing errors in Unique Molecular Identifiers to improve quantification accuracy." Genome research vol. 27,3 (2017): 491-499. Tian, Luyi et al. "scPipe: A flexible R/Bioconductor preprocessing pipeline for single-cell RNA-sequencing data." PLoS computational biology vol. 14,8 e1006361. Chen, Shifu et al. "fastp: an ultra-fast all-in-one FASTQ preprocessor." Bioinformatics vol. 34 (2018): i884-i880. Parekh, Swati et al. "zUMIs - A fast and flexible pipeline to process RNA sequencing data with UMIs." GigaScience vol. 7,6 (2018): giy059. Bray, Nicolas L et al. "Near-optimal probabilistic RNA-seq quantification." Nature biotechnology vol. 34,5 (2016): 525-7. Patro, Rob et al. "Salmon provides fast and bias-aware quantification of transcript expression." Nature methods vol. 14,4 (2017): 417-419. Srivastava, Avi et al. "Alevin efficiently estimates accurate gene abundances from dscRNA-seq data." Genome biology vol. 20,1 65. 27 Mar. 2019.

In step 235, the one or more processors may trim the received RNA-seq reads. In some embodiments, trimming comprises removing artificial sequences. For example, the one or more processors may scan for the longest contiguous run in the sequence with quality scores at or above the user-provided value, or may scan through reads from the 5' end and remove bases from the 3' end once the average quality score within the window drops below a user-specified value. In some embodiments, the one or more processors may account for both a high-quality and a low-quality score, and the one or more processors remove bases from the 3' end of reads that are below the high-quality score; once a base is encountered that surpasses the high-quality score, bases are retained so long as the bases between the low-quality score and high-quality score, as a fraction of total bases, does not rise above a default threshold. In some embodiments, trimming may be performed by Sickle, HTStream, BBduk, Trimmomatic, SolexaOA, or ConDeTri. Williams, Claire R et al. "Trimming of sequence reads alters RNA-Seq gene expression estimates." BMC bioinformatics vol. 17 103. 25 Feb. 2016.

In step 239, the one or more processors may deduplicate UMIs, such as those identifiers extruded in step 233. In some embodiments, deduplication comprises selecting a representative read. In some embodiments, deduplication comprises creating a consensus sequence. In some embodiments, deduplication of UMIs may be performed by a tool used for extraction of UMIs, e.g., as disclosed herein or known to one of skill in the art. In some embodiments, deduplication may be performed by UMIc, UMI-tools, or pRESTO. See, e,g., Tsagiopoulou, Maria et al. "UMIc: A Preprocessing Method for UMI Deduplication and Reads Correction." Frontiers in genetics vol. 12 660366. 28 May 2021.

In step 241, the one or more processors may perform a process of splitting. In some embodiments, step 241 comprises splitting the received RNA-seq reads received in step 231 by single-cell of origin determined by cell barcodes.

In step 245, the one or more processors may receive a set of RNA feature annotations and a set of genomic alignments in the known SAM (Sequence Alignment/Map) or BAM (Binary Alignment/Map) file formats. In some embodiments, the SAM or BAM file may include the name of a reference chromosome or contig to which the read has mapped, the start position of the read on the chromosome or contig, or the so-called Concise Idiosyncratic Gapped Alignment Report (CIGAR) string, giving the detailed alignment information including insertions, deletions, and other information, relative to the start position. In some embodiments, the RNA feature annotations may be stored in a single gene transfer format (GTF) file. In some embodiments, the genomic features may be specified in either GFF (General Feature Format) or SAF (Simplfied Annotation Format) format. Files in the SAF format may, in some embodiments, include five columns for each feature: feature identifier, chromosome name, start position, end position, and strand, because these five columns provide the minimal sufficient information for read quantification purposes. In either format, the feature identifiers are assumed to be unique, in accordance with commonly used Gene Transfer Format (GTF) refinement of GFF.

The RNA feature annotations (step 245) and the RNA-seq reads (step 231) correspond to the same reference genome. The reference genome is a set of reference sequences representing chromosomes or contigs. This enables annotation of the aligned RNA-seq reads to occur.

In step 237, the one or more processors may align the received and/or trimmed RNA-seq reads. In some embodiments, alignment comprises alignment of features in a RNA-seq read (from step 231) and features in an RNA feature annotation (from step 245).

In step 247, the one or more processors may perform a process of read-to-feature assignation for smallRNA. In some embodiments, the one or more processors may hash the sequence names from the RNA-seq read data into a hash table.

A hash table, in some embodiments, comprises a data structure that maps a "key" to a "value" using a mathematical function. For example, one or more processors may receive an input in the form of a feature from an RNA feature annotation (from step 245). The one or more processors may utilize a function associated with the hash table to map a received feature (a "key") to a string or a number (a "value"). In some embodiments, the one or more processors may execute a function on the received feature, such as dividing a numerical representation of the feature by a value and determining the remainder (also known as "hashing by division"). The remainder is a "value." The one or more processors may store the feature using the "value" as an index. A hash table enables quicker matching between a feature in the RNA-seq reads and the RNA feature annotations.

Instead of brute-force matching the feature annotation (step 245) with every possible aligned feature in the RNA-seq read (step 237), the one or more processors may use the hash function to determine the "value" corresponding to a feature annotation and determine whether the features stored at the "value" in the hash table match the feature. If there is a match, then the smallRNA feature has been found in the RNA-seq read.

In step 249, the one or more processors may perform a process of read-to-feature assignation for long exonic RNA. Step 249 may comprise, in some embodiments, matching long exonic RNA against features in the RNA-seq read data to determine a match. In some embodiments, the one or more processors may perform step 249 on RNA-seq read data that did not have an alignment overlapping in step 247. In some embodiments, the one or more processors may match long exonic RNA against features in the RNA-seq read data using the hash table discussed above.

In step 251, the one or more processors may perform a process of read-to-feature assignation for long intronic RNA. Step 251 may comprise, in some embodiments, matching long intronic RNA against features in the RNA-seq read data to determine a match. In some embodiments, the one or more processors may perform step 251 on RNA-seq read data that did not have an alignment overlapping in steps 247 or 249. In some embodiments, the one or more processors may match long intronic RNA against features in the RNA-seq read data using the hash table discussed above.

In step 253, the one or more processors may build a multi-mapping graph using the matched smallRNA and long exonic RNA (from steps 247 and 249, respectively).

In step 255, the one or more processors may detect MG (multi-graph) communities in the multi-mapping graph built in step 253. As discussed herein, MG communities, in some embodiments, may include groups of features with potential to produce identical or nearly identical RNA transcripts. In some embodiments, the one or more processors may generate a unique identifier and/or name for aggregating the corresponding alignments. The one or more processors may generate a table of MG communities linking each original feature received in step 245 with the resultant feature matching count matrix and metadata feature identifiers. In some embodiments, the table may include both aggregated features (which are collapsed following MG communities) and unique features (which remain unmodified). In some embodiments, the table may include the total number of alignments per feature.

In step 257, the one or more processors may generate an expression matrix.

The one or more processors may utilize the expression matrix generated in step 257 to output count matrix 259, feature metadata 261, and multi-map graph 263. In some embodiments, during count matrix building, the one or more processors may group longintronic RNA alignments according to the communities of features determined by the longexonic RNA graph for consistency in counting features.

Each row of count matrix 259, in some embodiments, corresponds to single features or to MG (Multi-Graph) communities aggregating several features. Each column of count matrix 259, in some embodiments, corresponds to one input file.

Feature metadata 261, in some embodiments, includes one or more of feature names matching row names in the count matrix, the counting round of hierarchical assignation and its configuration parameters, a flag designing whether a feature belongs to an MG community, or the feature biotype.

Multi-map graph 263, in some embodiments, includes a sparse adjacency matrix of links for each multi-mapping graph generated (smallRNA or/and longRNA) stored as a symmetric, integer, squared matrix. Each matrix element, in some embodiments, stores the number of alignments that multi-map to a pair of features (defined by row and column) and the diagonal contains the total number of alignments per feature. In some embodiments, the sparse adjacency matrix may be in MatrixMarket format.

FIG. 2C comprises aspects relating to FeatureCounts, discussed earlier in this disclosure, which are found in, for example, steps 231-245. One of ordinary skill will understand that other embodiments using different algorithms for read-to-feature assignation, such as HTSeq-count, may be used in place of these aspects.

### Example 1: MGcount Algorithm Overview

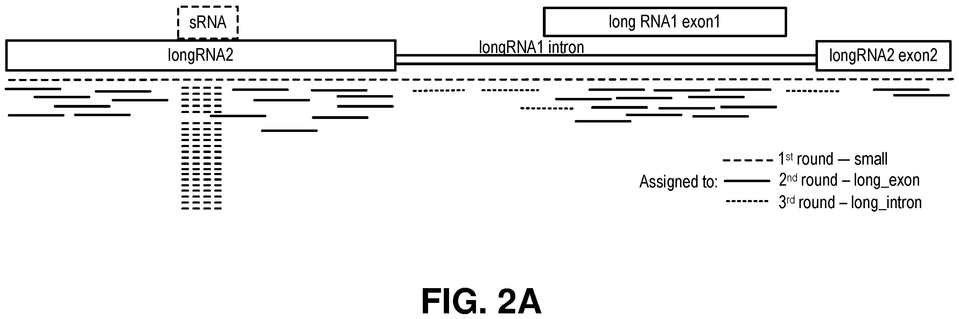

MGcount starts with a set of genomic alignments of RNA-seq reads (e.g., one BAM file per sample/cell) and a set of RNA feature annotations (e.g., stored in a single gene transfer format (gtf) file). It employs two strategies to quantify RNA features abundance in the sample, summarized in FIGs 2A-2C. (An example implementation of MGCount, consistent with the disclosed embodiments, is discussed below with respect to FIG. 2C.) First, a hierarchy based on transcript body length is used to solve multi-overlapping ambiguities across RNA biotypes during alignment-to-feature assignment. Hence, all reads with at least one alignment overlapping with an annotation in the current round can only be assigned to features in such round and skipped in subsequent rounds. Second, a multi-mappers-based data-driven approach is applied to recognize meaningful groups of sequence-related RNA features that are subsequently defined as new aggregated features for a more confident quantification. MGcount, in some embodiments, built on top of FeatureCounts, a computationally efficient counting software. FeatureCounts is a read summarization program that implements highly efficient chromosome hashing and feature blocking techniques suitable for counting reads generated from RNA or genomic DNA sequencing experiments. FeatureCounts performs precise read assignment by comparing mapping location of every base in the read or fragment with the genomic region spanned by each feature. It takes account of any gaps (insertions, deletions, exon-exon junctions or fusions) that are found in the read. It calls a hit if any overlap (1 bp or more) is found between the read or fragment and a feature. Liao, Yang et al., featureCounts: an efficient general-purpose program for assigning sequence reads to genomic features, Bioinformatics, Volume 30, Issue 7, 1 April 2014, Pages 923-930.

### Hierarchical assignation

MGcount hierarchically assigns reads to annotated genomic features in three predefined sequential rounds, named "small," "long_exon," and "long_intron". First, in the "small" round, alignments are assigned to smallRNA biotypes (microRNA, piRNA, snRNA, rRNA, snoRNA, tRF, YRNA and vaultRNA, among others) and prioritized in situations where an alignment overlaps both a smallRNA and a longRNA (mRNA or lncRNA) feature (FIG. 2A). Without being bound by theory, this can be justified by the length disparity between them, i.e., a read overlapping both a smallRNA and a longRNA feature has more likely been generated from a smallRNA than a much longer longRNA transcript. As shown in FIG. 3A-3D, smallRNAs constitute the majority of overlapping cases. In cases where reads are present throughout the long-RNA locus and when the majority of or all reads might have come from the long RNA, assigning overlapping reads to the small RNA will impact the expression quantification of the long RNA only marginally. The second round in the hierarchy assigns reads to long RNA exons. In a final round, MGcount assigns remaining reads to longRNA introns (which by default are defined by the full gene body coordinates), that may be abundant in RNA-seq data due to unspliced transcripts. Without being bound by theory, these second and third rounds are justified since unspliced transcripts (with introns) are short-lived compared to mature transcripts (without introns). Hence, it is more likely that a mature transcript is detected in situations where an exon of a longRNA overlaps with an intron of another longRNA.

At each round of the hierarchical assignation, alignment-feature assignation pairs are determined with FeatureCounts restricted to the designated subset of the gtf annotated features. Each round can be configured by the user through five arguments (Table 1). These are (1) "feature", the annotation entry type considered for read-to-feature assignation; (2) "feature_output", the annotations attribute for which feature abundance will be reported; (3) "feature_biotype", the annotations attribute defining the feature biotype, which determines membership to a round; (4) "min_overlap", which defines the minimum overlapping fraction of the read required to assign an alignment to an annotation; and (5) "ml_flag", to activate or disable communities detection and feature aggregation for each round (next section). MGcount incorporates a list linking the common biotypes to the small/long category that is used, at each round, to subset annotations according to the "feature_biotype" variable. This list can be customized by the user. All arguments in Table 1 can be independently configured for each round, which facilitates dealing with smallRNA and longRNA features annotated under different columns in the gtf.

**Table 1 Configurable parameters and default values for each read-to-feature assignation round. LongRNA introns are by default defined by the full gene body coordinates.**

| Round | feature | feature_output | feature_biotype | min_overlap | ml_flag |
|---|---|---|---|---|---|
| small | transcript | transcript_name | transcript_biotype | 1 | True |
| long_exon | exon | gene_name | gene_biotype | 1 | True |
| long_intron | gene | gene_name | gene_biotype | 1 | True |

### Multi-mapper communities detection

MGcount exploits graph structures to model resemblances between annotated features, in its potential to produce the same transcript, from real evidence coming from RNA-seq data. MGcount builds a directed weighted graph G=(V,E) where each vertex from the set of vertices V is a feature (as defined by the feature_output parameter) with a weight equal to the log-transformed number of assigned alignments. Directional edges (E) connect features for that share multi-mapping reads. Each directional edge is assigned a weight that is proportional to the ratio of multi-mapping reads between the two connected vertices normalized by the total number of reads assigned to the source vertex. (FIG. 2B). Graphs are generated independently for smallRNA and longRNA, using the full pool of alignments from all input samples.

Next, highly related features, where reads systematically multi-map, are grouped together by minimizing an objective function known as the map equation with the communities detection approach described by Rosvall et al., 2008. Rosvall, Martin, and Carl T Bergstrom. "Maps of random walks on complex networks reveal community structure." Proceedings of the National Academy of Sciences of the United States of America vol. 105,4 (2008): 1118-23. See also Rosvall, Martin et al. "The map equation." European Physical Journal: Special Topics 178(1), 13-23 (2009). The map equation models the duality between compressing and detecting regularities (i.e., modules of highly dense interconnected vertices in the graph). The Rosvall map equation formulates the description length of an infinite random walk trajectory along the graph as a function of its topology. The walk description is defined as a sequence of two bitcode dictionaries, wherein vertices are tagged to describe within community movement and communities are tagged to describe inter-community movement. The larger the weight of a connection between two vertices, the more it is visited by a random walk. Thus, the goal of the map equation is to minimize the description length by identifying the communities for which the random walk stays the maximum within and moves the minimum between. Resultant graph communities represent groups of features with potential to produce identical or nearly identical RNA transcripts, hereafter referred to as MG communities (Multi-Graph communities). Each MG community is given an identifier and a new name that is subsequently used to aggregate the corresponding alignments.

The Rosvall et al., 2008 algorithm defines a small "teleporting" probability connecting every vertex in the network to guarantee a unique steady state distribution in directed networks. MGcount assigns non-uniform teleporting probabilities proportionally to the vertices weight, correcting for over visit frequencies to features with fewer alignments. MGcount ignores weak edges during map equation optimization based on a high and a low threshold, predefined as 0.75 and 0.01 respectively, that can be defined by the user and are common for all biotypes. For the longRNA graph, edges below the high threshold are ignored. For uniformity, intronic features are analogously aggregated as exonic feature communities. For smallRNA, the threshold closer to the graph weights' first quantile is employed for each biotype to prevent over-fitting (splitting of large densely connected communities and merging of small loosely connected communities) according to the weights' distribution of a graph.

MGcount assigns a biotype to each detected community based on its features. The label "hybrid" is used when an MG community contains features from different biotypes, which might occur for longRNA or when the .gtf file contains duplicated named entries (with the same identifier under feature_output) but with different biotypes. In addition, any biotype prevails over "pseudogene" features. Hence, when a community contains pseudogene features and non-pseudogene features, a preference is made for the latter to prioritize for the active biotypes. In the smallRNA graph, although MGcount creates one graph for the total set of features, MG communities are independently detected per biotype.

### Count matrix building

MGcount generates an output expression matrix for each hierarchical assignation round and concatenates them in a single output matrix. For each read, each alignment first gets a "fractionated count" of 1/N, where N is the number of multi-mappers or multi-overlappers that survived the hierarchical assignment because they aligned to two features from biotypes in the same round. Next, counts for annotations that have been aggregated together in a community by the map equation are summed up (communities become newly defined features). In this way, the systematic ambiguity in multi-mapping reads collapses into a single MG community while the remaining signal is reported as fractionated counts.

### Software execution - Inputs and Outputs

To run MGcount, three inputs are required: a .txt file listing the paths to the .bam alignment input files a .gtf (annotations) file and the output directory path. Optional arguments may be added to configure the tool according to data parameters such as single/paired sequencing data, stranded/unstranded library preparation, or the configuration arguments summarized in Table 1. Further, to speed-up computation time a fixed number of random sub-sampled alignments per sample can be set to build the graph.

At the end of its execution, MGcount provides the following outputs:
- A count matrix where each row corresponds to a feature as defined by feature_output (either single features or MG communities aggregating several features) and each column corresponds to one input .bam file.
- A feature metadata table reporting: feature names matching row names in the count matrix, the counting round of hierarchical assignation and its configuration parameters, a flag designing whether a feature belongs to an MG community, and the feature biotype.
- A sparse adjacency matrix for each multi-mapping graph generated (smallRNA or/and longRNA) stored as a symmetric, integer, squared matrix. Each matrix element stores the number of alignments that multi-map to a pair of features (defined by row and column) and the diagonal contains the total number of alignments per feature.
- A table of MG communities linking each original feature in the gtf file with the resultant feature matching count matrix and metadata feature identifiers. It includes both unique features (which remain unmodified) and aggregated features (which are collapsed following MG communities). Also, the table stores the total number of alignments per feature.

### Example 2: Libraries Preparation

Libraries for *Arabidopsis* roots, Mouse Liver, Nematode, Human Brain and K652 human cells were prepared in duplicates with the D-Plex Small RNA-seq Kit for Illumina (Diagenode Cat#C05030001) which employs polyA tailing and template-switching. Further, two additional libraries for each human template (Human Brain and K562 cells) were prepared after enriching for smallRNA content with MiRNeasy tissue/cells advanced kit (Qiagen Cat#217684). Sequencing was performed on Illumina technology under the following parameters: SE75 for Human Brain, SE50 for K562 cells samples and SE100 for the rest of samples (Nematode, Arabidopsis, and Mouse). All the libraries were prepared and sequenced in duplicates.

### Example 3: NGS Pre-Processing

Unique molecular identifiers (UMI) of length 12 were extracted from each read with fumi_tools v.0.18.1. Reads were trimmed for Illumina adapters and polyA tails using cutadapt v3.0 with arguments "-trim-n -match-read-wildcards -u 16 -n 4 -a AGATCGGAAGAGCACACGTCTG -a AAAAAAAA -a GAACTCCAGTCAC -e 0.2 - nextseq-trim 20 -m 15" and subsequently aligned to the reference genomes using STAR v2.7.0d with arguments -outFilterMultimapScoreRange 0 -outFilterMultimapNmax 50 - outFilterMismatchNoverLmax 0.05 -outFilterMatchNmin 15 -outFilterScoreMinOverLread 0 -outFilterMatchNminOverLread 0 -alignIntronMax 1 -readFilesCommand zcat -outSAMtype BAM SortedByCoordinate -outSAMunmapped Within". Finally, PCR clones were removed by UMI-based deduplication using fumi_tools.

### Example 4: qPCR

A subsequence from 20 different smallRNA targets was selected to design a miRCURY LNA custom PCR plate of 96 wells (Cat #339330) so that each plate contained 4 wells measuring the same target. Two plates were employed to quantify targets expression in quadruplicates for Human Brain and K562 samples. Relative abundance of each target was estimated by normalizing Cq values obtained with RT-qPCR for each target with respect to SNORD49A.

### Example 5: Database Integration

To generate the gtf files integrating annotations from multiple databases Ensembl was considered the primary annotations source. Next, annotations from different databases were appended after curation and reformatting to an Ensembl-like structure. piRNA and siRNA were reformatted as three gtf rows encoding to an Ensembl-like single gene-transcript-exon structure. miRNA precursor and tRNA features were reformatted to Ensembl-like gene entries while the miRNA mature features and tRNA fragments (tRF) were integrated as transcript and exon features with gene_id and gene_name given by its precursor. Since RNACentral aggregates multiple annotation sources, annotations from this database (piRNA in Mouse and siRNA in Arabidopsis) were semi-automatically curated with a custom script detecting groups of annotations overlapping by more than half of the annotation body (considered redundant) and selecting the annotation with median coordinates within the overlapping set for integration. According to the species, annotations include: *A. thaliana:* Ensembl, miRBase (microRNA) and RNACentral (siRNA); *H. Sapiens* Ensembl, DASHR (piRNA and tRF) and miRBase (microRNA); *M. musculus:* Ensembl, miRBase (microRNA) and RNAcentral (piRNA); *C*. *elegans:* Ensembl and miRBase (microRNA).

### Example 6: Single Cell total-RNA-seq Data Analysis

Single-cell dataset count tables were pre-processed with the Seurat package. Hao, Yuhan et al. "Integrated analysis of multimodal single-cell data." Cell vol. 184,13 (2021): 3573-3587.e29. Cells with more than 2000000 counts or less than 2000 features in the reference pipeline were filtered out from both reference table and MGcount table. Isakova, Alina et al, "Single cell profiling of total RNA using smart-seq-total." bioRxiv (2020). Counts were log-normalized with a scale factor of 1000000; centered and scaled by the vector of variable features detected with the variance stability transformation method with default parameters. Principal Component Analysis was performed on the subset of smallRNA and longRNA exonic counts. First and second Principal Components were employed for visualization (FIG. 8A). Differentially expressed smallRNA features were detected with a Wilcoxon Rank Sum test (adjusted p-value below 0.05) between cell populations.

### Example 7: Validation of MGcount

We generated and analysed RNA-seq libraries across 4 well-studied species, namely, *A. thaliana, H. sapiens, M. musculus* and *C*. *elegans* to characterize the behaviour of multimapping and multi-overlapping reads and the effect of MGcount quantification over distinct biotypes. We further evaluated MGcount performance in two different human RNA templates (K562 cell line and human brain) and validated the accuracy of the estimated counts for a set of 20 smallRNA markers with RT-qPCR. Finally, we tested MGcount on a publicly available total-RNA-seq dataset with single-cell resolution. Isakova, Alina et al, "Single cell profiling of total RNA using smart-seq-total." bioRxiv (2020).

### Example 8: Hierarchical Assignation Resolves smallRNA-longRNA Multi-Overlappers

In order to assess the potential impact of overlapping features from different biotypes on RNA-seq analysis, we explored their overlap frequencies. For this task, we used as a reference a customized GTF file that integrates several databases for the following species: *H*. *sapiens, M. musculus, C. elegans* and *A. thaliana).* FIGs. 3A-3D present the frequency of overlaps by biotype combination. In all organisms, the most overlaps occur between longRNA and smallRNA transcripts, which significantly differ in transcript body length, supporting the rational for the two-step hierarchy employed by MGcount. Triple overlaps commonly involve a longRNA and two smallRNA biotypes. For the latter, MGcount would fractionally split the multi-overlapping alignment within a same counting round. Patterns show consistency across species.

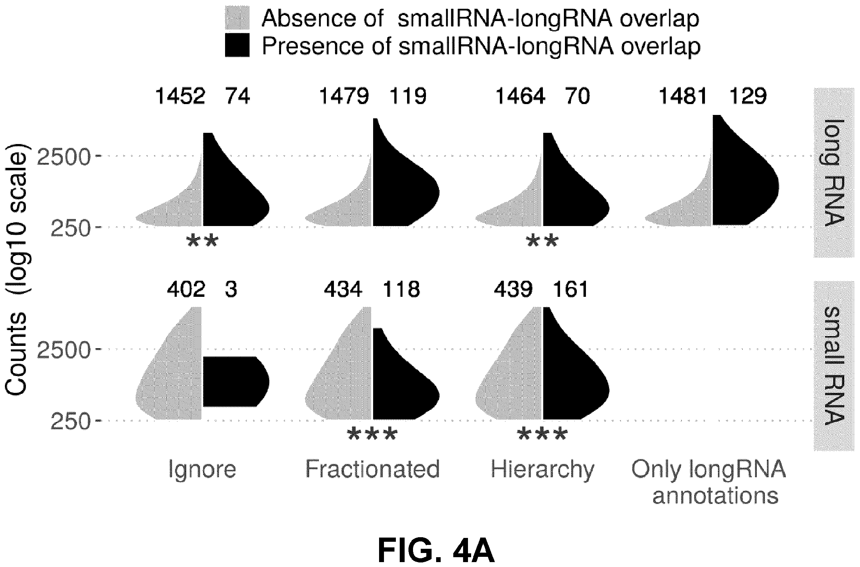

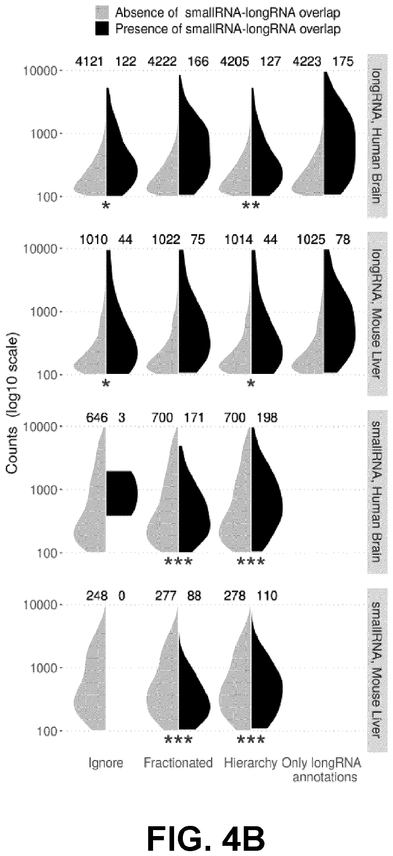

Next, we evaluated the effect of the hierarchical assignation of reads on resolving smallRNA-longRNA multi-overlapping ambiguities. FIGs. 4A-4B compares the similarity of the counts distribution in absence and presence of smallRNA-longRNA feature overlaps using the Two-One-Sided Tests (TOST) approach, using four different multi-overlap quantification strategies: discarding overlapping reads; counting overlaps as fractions (1 divided by the number of overlaps); MGcount's hierarchical strategy (assigning counts to smallRNAs first, fractionated in the case of other types of overlap); and considering only longRNA annotations.

Results show that ignoring smallRNA annotations in total-RNA-seq data led to an inflated estimation of certain longRNA transcripts, due to the incorrect assignment of reads as longRNA that instead originated from smallRNA transcripts embedded in longRNA. A smaller inflation in counts was observed when partially counting in fractionated mode. Both the hierarchical assignation and discarding multi-overlapping reads approaches resulted in similar distributions, suggesting that multi-overlapping alignments are mainly associated with smallRNA reads and not longRNA reads (that spread over a long gene body). These results suggest that the discarding multi-overlappers strategy and the hierarchical assignation strategy are adequate to quantify longRNA biotypes.

However, the discarding multi-mappers approach was not adequate for smallRNA quantification. Instead, discarding multi-overlapping reads resulted in discarding smallRNA transcripts embedded within (i.e., fully overlapping with) a longRNA. By contrast, hierarchical assignation and fractionated counting produced comparable distributions. There, were, though, observed drawbacks to the fractionated counting approach, which showed a slight mismatch of overlapping smallRNAs distribution towards lower expression values. This suggests again that the fractionated counting approach can mis-quantify some smallRNAs as longRNA.

In summary, the hierarchical assignment strategy, e.g., as performed using MGcount, was the only strategy of the four tested strategies that produced equivalent distributions of counts with and without overlapping events for both longRNA and smallRNA transcripts. These findings support the rationale that reads that fully map to a smallRNA that overlaps a longRNA most likely belong to the smallRNA, and demonstrates that MGcount's hierarchical assignation approach successfully resolves longRNA-smallRNA overlapping ambiguities.

### Example 9: MG Communities Detection is Robust at both Intra-Sample and Inter-Sample Levels

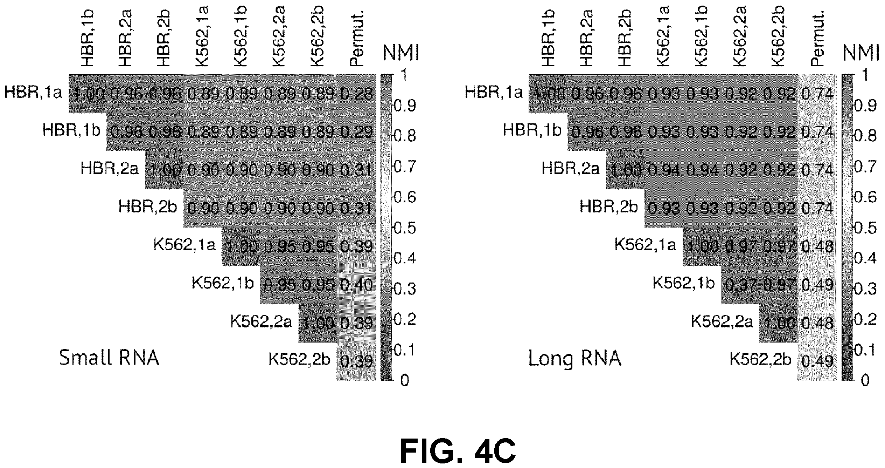

To evaluate the robustness of the MG community detection, we compared the communities detected with different seeds (for random-number generators) on the same and on different total-RNA-seq input datasets. We separately processed each replicate of Human Brain and K562 libraries twice and computed the Normalized Mutual Information (NMI) between partitions of commonly clustered features (FIGs. 4C-4F). Each solution was also compared to randomized MG communities obtained by permuting the grouping labels of all vertices in the graphs and averaging the resulting NMI. Both longRNA and smallRNA partitions were nearly identical for the two different runs within the same input dataset (NMI = 1), demonstrating stable convergence and minimal variability due to the algorithm's stochastic component. Communities across biological replicates showed high similarity in both Human Brain and K562 libraries and comparison between the two templates exhibited only a small reduction of the similarity ofsolutions. This analysis shows high reproducibility between MG communities obtained from RNA-seq libraries prepared under similar technical conditions, independent of the RNA origin, and demonstrates the robustness of MGcount's community detection.

### Example 10: Validation of Expression Quantification via RT-qPCR

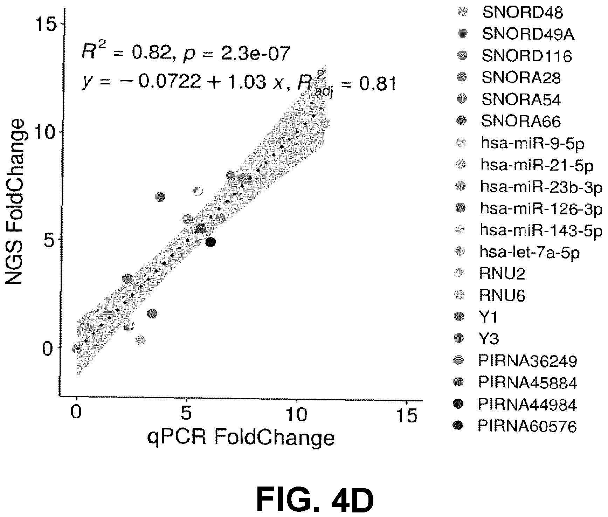

To independently check the accuracy of the quantification between smallRNAs of different biotypes, we compared the total-RNA-seq MGcount expression levels of human brain and K562 libraries with estimates of expression levels from RT-qPCR for 20 smallRNA markers with different multi-loci profiles (FIG. 4D). All transcript abundances were normalized by SNORD49A, which was highly expressed in both samples. The linear regression showed good adjustment in modelling the fold change concentration relationship between the Human Brain and K562 libraries, independently measured with total-RNA-seq libraries and with RT-qPCR.

### Example 11: Multi-mapping Graphs Capture RNA Loci Structure and Identify Feature Communities Coding for Sequence-Similar Transcripts

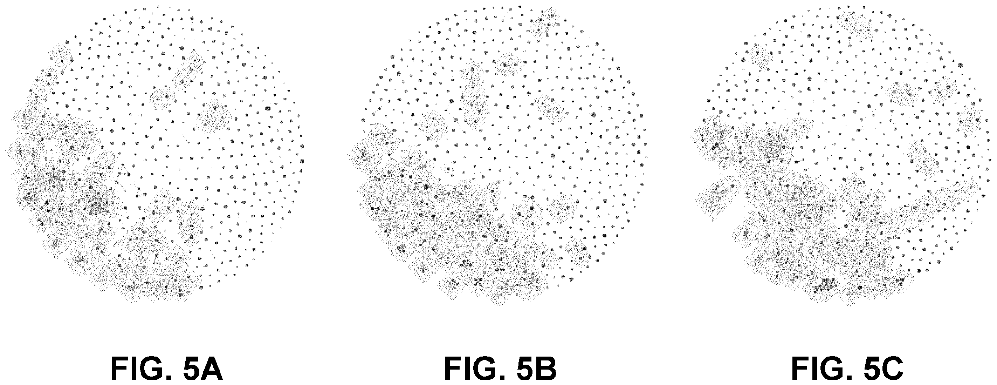

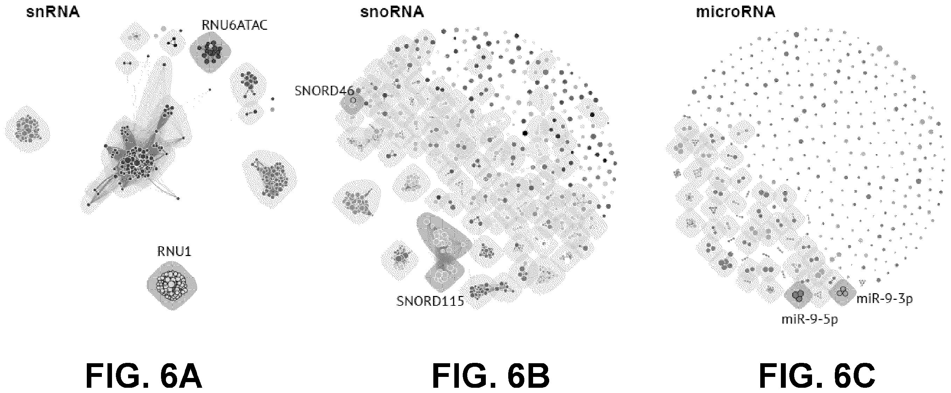

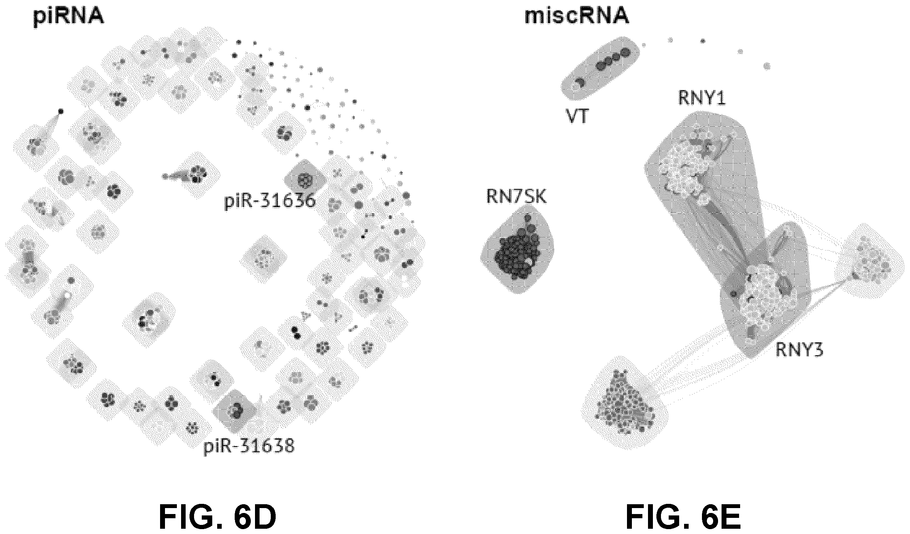

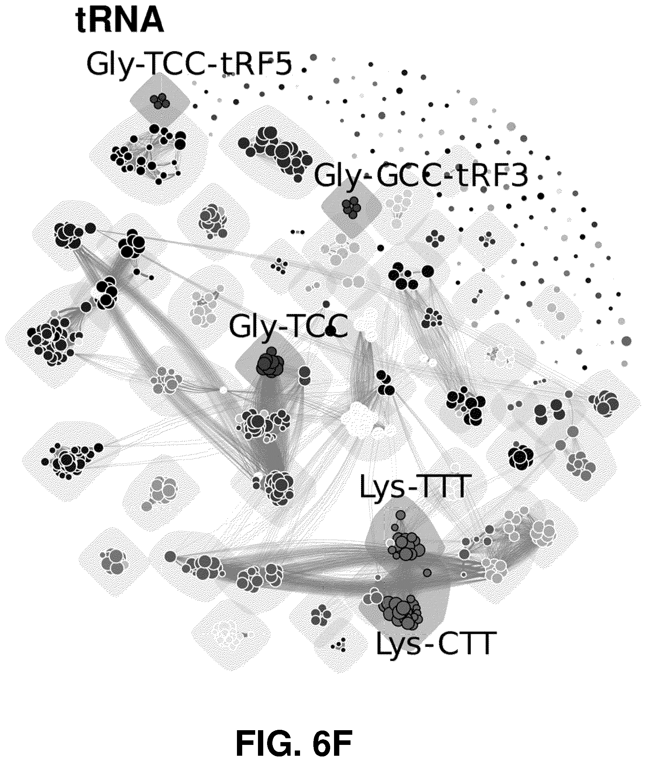

FIGs. 5A-C and 6A-6F present visualizations of the graphs obtained from the pool of human libraries (K562 cell line and Human Brain) in which vertices are annotated features from the gtf, edges represent shared multi-mapping reads between them, and gray areas delineate the MG communities detected by MGcount. For longRNA, MGcount detected 2946 MG communities, while 26087 features remained as individually quantified features. For simplicity, FIG. 5A-5C display three sub-graphs extracted by randomly sub-sampling 300 features, either individually or MG communities. The color/shade of vertices indicate alignment biotype (protein-coding, pseudogene, long non-coding RNA). As expected, most protein-coding features remained single (blue vertices in color version of drawing), as they mainly transcribe from individual loci and are aligned by uniquely-mapping reads. Also, MGcount detected large communities containing pseudogenes (yellow vertices in color version of drawing) and protein-coding genes. In these cases, MGcount avoids false expression of pseudogene inactive loci copies and subsequent under expression estimation of active genes.

FIGs. 6A-6F show the graphs for the most abundant smallRNA biotypes generated by MGcount. In general, our results show very different graph topologies for each biotype. In most of the cases, we detected that features grouped together in each MG community have similar annotations as given by the Hugo Gene Nomenclature Committee (HGNC), which establishes a standard nomenclature framework for RNA classes within major smallRNA biotypes in Human species. Wright, Mathew W, and Elspeth A Bruford. "Naming 'junk': human non-protein coding RNA (ncRNA) gene nomenclature." Human genomics vol. 5,2 (2011): 90-8. Seal, Ruth L et al. "A guide to naming human non-coding RNA genes." The EMBO journal vol. 39,6 (2020): e103777. For example, the snRNA graph has a few large MG communities that correspond to the different well-known spliceosomal sub-units, present in multiple copies. Guiro, Joana, and Shona Murphy. "Regulation of expression of human RNA polymerase II-transcribed snRNA genes." Open biology vol. 7,6 (2017): 170073. This is also the case of most abundant RNA classes within miscRNA, that comprises various RNA biotypes as 7SKRNA or YRNA sub-units with their respective retrotransposition-derived pseudogenes spread over all the genome. Perreault, Jonathan et al. "Retropseudogenes derived from the human Ro/SS-A autoantigen-associated hY RNAs." Nucleic acids research vol. 33,6 2032-41. 7 Apr. 2005. These are repeatedly multi-mapped by reads, and therefore grouped together in large communities. Contrarily, most microRNA annotations stayed single given its unique loci structure while the few loci sets transcribing the same microRNA were aggregated. Here, we quantified microRNAs from its 3p and 5p mature transcript form annotations which consistently got aggregate with themselves. We observe more heterogeneous profiles with snoRNA, where a few large communities are detected, while other snoRNA features have either 1 locus or a small number of related loci. piRNA exhibit compact communities in relation with genomic regions where piRNA sequences reside. Siomi, Mikiko C et al. "PIWI-interacting small RNAs: the vanguard of genome defence." Nature reviews. Molecular cell biology vol. 12,4 (2011): 246-58. For tRNA derived fragments, communities mainly follow amino acid type and fragment position within the tRNA precursor.

All smallRNA graphs positively agree with existing knowledge encoded in the HGNC gene symbol annotations. MGcount could be thus safely applied to detect communities in species with minimal or poor annotations. Already in our results, some MG communities integrate features not following the HGNC annotations system, usually associated to computationally predicted annotations (grey nodes in FIGs. 6A-6F), with other well-characterized smallRNA. For example, SNORD46 (Chr1:44,776,492-44,776,589) was clustered together with AC009365.1 (Chr7:132,753,023-132,753,126), a repeated loci diverging only in 15 out of 104 nucleotides. This points to the potential application of MGcount in assigning computational predictions to their corresponding RNA family during quantification.

### Example 12: MGcount Reduces Multi-Mapping and Multi-Overlapping Ambiguity

FIG. 7A (and left-most data column of FIG. 7D) shows the impact of different raw counting rules (discard multi-assignments, count all multi-assignments, fractionally count multi-assignments) in the total read counts per biotype in different organisms. The quantification of protein-coding genes is little impacted by the selected strategy given lower multimapping situations. However, while counting only uniquely mappers results in a loss of information, counting all multi-mappers and multi-overlaps inflates the expression values (log scale) in non-coding features. MGcount provides more counts for smallRNA biotypes than fractionated counting, as a consequence of the hierarchical assignation step. Also, pseudogene counts are lower because several pseudogenes assignments cluster together with protein-coding assignments within the MGcount algorithm. These results and comparisons highlight the sensitivity of RNA expression quantification to the selected multi-assignment handling approach, particularly when quantifying non-coding transcripts.

In FIGS. 7B-7C (and the middle and right-most data columns of FIG. 7D, left-hand barplots show the fraction of reads by number of multi-overlappers and multi-mappers in the total-RNA-seq libraries analysed. Right-hand-barplots show how multi-feature read assignment fractions get reduced by MGcount.

On one hand, multi-overlaps occur much infrequently given the hierarchical assignment of smallRNA followed by longRNA. On the other hand, the multi-graph strategy collapses features where reads systematically multi-map, converting them into single features. Multi-mapping and multi-overlapping fractions show consistent patterns across species over RNA biotypes. The major proportion of multi-overlapping alignments result from smallRNA embedded within a protein-coding or a long noncoding, as FIGs. 3A-3D show. Alignments exhibiting triple overlaps result mostly from reads mapping to genomic regions where a short smallRNA arises from middle-size smallRNA simultaneously embedded within a longRNA, as is the case for snoRNA derived microRNAs or piRNAs. Taft, Ryan J et al. "Small RNAs derived from snoRNAs." RNA (New York, N.Y.) vol. 15,7 (2009): 1233-40. Scott, Michelle S, and Motoharu Ono. "From snoRNA to miRNA: Dual function regulatory non-coding RNAs." Biochimie vol. 93,11 (2011): 1987-92. Falaleeva, Marina, and Stefan Stamm. "Processing of snoRNAs as a new source of regulatory non-coding RNAs: snoRNA fragments form a new class of functional RNAs." BioEssays : news and reviews in molecular, cellular and developmental biology vol. 35,1 (2013): 46-54. He, Xin et al. "An Lnc RNA (GAS5)/SnoRNA-derived piRNA induces activation of TRAIL gene by site-specifically recruiting MLL/COMPASS-like complexes." Nucleic acids research vol. 43,7 (2015): 3712-25. In these cases, MGcount fractionally attributes the read to both smallRNAs (see, e.g., FIG. 7B right). As for multi-mapping alignments patterns, reads mapping to smallRNA show higher fractions. This is explained by their high copy number in the genome, mainly arisen from retro-transposition events. Weber, Michel J. "Mammalian small nucleolar RNAs are mobile genetic elements." PLoS genetics vol. 2,12 (2006): e205. An especially prominent effect was observed in mid-sized smallRNAs like tRNA and snRNA (see, e.g., FIG. 7C). Boivin, Vincent et al. "The cellular landscape of mid-size noncoding RNA." Wiley interdisciplinary reviews. RNA vol. 10,4 (2019): e1530. Doucet, Aurélien J et al. "U6 snRNA Pseudogenes: Markers of Retrotransposition Dynamics in Mammals." Molecular biology and evolution vol. 32,7 (2015): 1815-32. The same holds for a small subset of snoRNA, which links to the fact they are encoded in tandem copies while others are produced from a unique locus. Bratkovič, Tomaž et al. "Functional diversity of small nucleolar RNAs." Nucleic acids research vol. 48,4 (2020): 1627-1651. With consistency between species, the observed patterns highlight the magnitude of the biological signal encoded in reads mapping to more than one RNA feature. We find that a direct relation exists between the multi-mapping or multi-annotated patterns and the biological nature of different transcripts, justifying the need of an adaptive strategy such as MGcount that treat multi-mapping as an aggregated signal rather than as an ambiguity or artifact.

### Example 13: MGcount Detects Expression of Cell-Specific Non-Coding RNA Communities

To evaluate the software performance with single-cell resolution, we ran MGcount on a public single-cell total-RNA-seq dataset consisting of 637 cells from three human cell-lines (Dermal Fibroblasts, HEK293T cells and MCF7 cells; FIG. 8A). We performed differential expression analysis on smallRNA features between the three cell populations (Wilcoxon Rank Sum test, adjusted p-value below 0.05) using count tables in which features were aggregated in MG communities (MGcount) and count tables obtained with the reference pipeline described in Isakova, Alina et al, "Single cell profiling of total RNA using smart-seq-total." bioRxiv (2020).. The gtf integrating several databases was used in both cases. Our results show that the average log fold-change of differentially expressed smallRNA features detected by MGcount is larger than that of the reference pipeline, indicating that the aggregation of the multi-mapping signal helps detecting stronger effects. With the reference pipeline, we detected 397 statistically significant small-RNA-annotated features at an average log2 fold-change above 0.5, whereas with MGcount, we detected 179 features (out of which 94 were communities of multiple, originally annotated features, and 85 were individual features) (Fig. 5a). These correspond to 1167 of the originally annotated features. Remarkably, by setting the log2 fold-change threshold at 2.5, MGcount detects 28 significant features (including 10 MG communities). These equals to 132 features while with the reference pipeline only 63 are detected. Some MG communities were predictive markers of specific cell types; e.g., the SNORD114 loci tandem cluster located within the human 14q32 locus for dermal fibroblasts (FIGs. 9B-9C) and piR-36011 cluster for HEK293T cells (FIGs. 9B, 9D). These results show that MGcount recovers biologically meaningful information from multi-assigned reads at single-cell resolution.

### Example 14: Discussion of Validated MGcount Approach

RNA-seq reads may align equally well to multiple genome positions, where at the same time, more than one RNA transcripts may originate. While traditional pipelines focusing on protein-coding transcripts commonly discarded these reads, the size of biological information encoded in such ambiguous alignments is very large in datasets comprising noncoding RNA. (see, e.g., FIGs. 7B-7C). To date, different Bioinformatic tools have proposed solutions to decide how an ambiguous read should contribute to the expression estimation of the multiple transcripts where it aligns. However, to our knowledge, existing solutions have focused on particular biotypes or model-species and make assumptions that are not met by all totalRNA-seq datasets, which demand flexible approaches to simultaneously quantify any transcript.

As shown by at least the Examples provided herein, MGcount provides a flexible quantification framework for transcriptome-wide scientific studies that interrogate heterogeneous RNA-seq datasets comprising several non-coding biotypes. First, a hierarchical assignation workflow resolves frequent overlaps of smallRNAs embedded in longRNA loci and allows to distinctly quantify longRNA spliced and unspliced features. Then, to quantify multi-mappers, instead of making a choice about which features a read contributes to (guess the true alignment, weight several alignments, etc.), families of features that are similarly transcribed are detected and aggregated in MG communities. With this approach, we "zoom out" from genomic loci resolution and quantify aggregated communities of annotations while gaining in confidence that the given read is originated from the community of annotations where it is assigned. Concurrently, this approach allows defining a meaningful output-level for quantification in a data driven manner, collapsing these repeated loci that are associated to the same RNA product. This solution may also be used to quantify poorly annotated transcripts as a community instead of diluting them as several "unknown features," each of which may be at a low level of expression. MGcount preserves the multi-mapping information for downstream analyses, allowing better quantification of smallRNA biotypes and reducing assignation errors and biases associated to multi-alignments handling premises that do not suit all biotypes structure. While the concept of gene merging had been previously suggested for the study of mRNA in [32], here we provide a novel method that first recognizes the multiple-loci meaningful groups by treating alignments-feature assignation data as a graph which allows to discern between systematic multi-mappers (used to define aggregated features) and residual multi-mappers (ignored in feature aggregation and weighted quantified) by means of a robust community detection approach. We find that the graph provides an integrative representation of transcripts multi-loci profiles that enhances interpretability. For instance, this may be used as an exploratory tool to characterize computationally predicted annotations in less studied species.

Like all RNA-seq quantification tools, MGcount quantification is limited by the scope of annotations. Part of the non-coding transcriptome is still undiscovered and unannotated (even in well-studied species). Boivin, Vincent et al. "Reducing the structure bias of RNA-Seq reveals a large number of non-annotated non-coding RNA." Nucleic acids research vol. 48,5 (2020): 2271-2286. Yet, in the smallRNA study field, algorithms have been developed to predict and annotate transcript loci based on RNA conservation. For instance, Rfam is a database resource comprising mathematical models of different RNA classes capturing sequence similarity across species and molecular secondary structure. Kalvari, Ioanna et al. "Rfam 13.0: shifting to a genome-centric resource for non-coding RNA families." Nucleic acids research vol. 46,D1 (2018): D335-D342. The models allow to de-novo annotate regulatory RNA with software tools on a new genome, as possible with other published smallRNA prediction algorithms [60]. Eddy, S R, and R Durbin. "RNA sequence analysis using covariance models." Nucleic acids research vol. 22,11 (1994): 2079-88. Nawrocki, Eric P, and Sean R Eddy. "Infernal 1.1: 100-fold faster RNA homology searches." Bioinformatics (Oxford, England) vol. 29,22 (2013): 2933-5. Zhang, Yi et al. "A Review on Recent Computational Methods for Predicting Noncoding RNAs." BioMed research international vol. 2017 (2017): 9139504. With the ability to computationally annotate regulatory small RNA, MGcount fills a gap by revealing the structure of predicted annotations from experimental evidence coming from RNA-seq data. MGcount thus may have applicability not only in expression quantification but to uncover smallRNA genomic structure profiles.

### Conclusion

MGcount is a novel RNA-seq quantification tool that combines two strategies to quantify ambiguous alignments in an adaptive data-driven manner. Its approach is suitable for wide full-transcriptome analysis estimating the concentration of protein-coding, long noncoding, and small non-coding transcripts in both its precursor and processed forms simultaneously. This allows a wider and more inclusive interrogation of total-RNA-seq data. MGcount models read-to-feature alignment ambiguities with biotype-specific graphs that are employed to detect communities of sequence-similar transcripts. Besides quantification of transcript expression, such graphs constitute a powerful computational tool to inspect the structure of multi-loci copies from sequencing data, enhancing the interpretability of results. Given its flexibility, MGcount is especially useful for the study of the interplay between protein-coding and regulatory RNAs in biological processes, even in less characterized species, at both bulk and single-cell resolution.

While the present disclosure has been shown and described with reference to particular embodiments thereof, it will be understood that the present disclosure can be practiced, without modification, in other environments. The foregoing description is not exhaustive and is not limited to the precise forms or embodiments disclosed. Modifications, combinations, and adaptations will be apparent to those skilled in the art from consideration of the specification and practice of the disclosed embodiments. Moreover, while illustrative embodiments have been described herein, the scope of any and all embodiments having equivalent elements, modifications, omissions, combinations (e.g., of aspects across various embodiments), adaptations and/or alterations as would be appreciated by those skilled in the art based on the present disclosure.

Computer programs based on the written description and disclosed methods are within the skill of an experienced developer. Various programs or program modules can be created using any of the techniques known to one skilled in the art or can be designed in connection with existing software. For example, program sections or program modules can be designed in or by means of .Net Framework, .Net Compact Framework (and related languages, such as Visual Basic, C, etc.), Java, C++, Objective-C, HTML, HTML/AJAX combinations, XML, or HTML with included Java applets.

## Claims

1. A computer-implemented method for quantification of RNA expression from RNA sequence reads, comprising:
(a) hierarchically assigning RNA sequence reads from at least one cell to RNA feature annotations according to RNA transcript length, to yield feature-read pairs assigned as small RNA, long exonic RNA, or long intronic RNA, and
(b) detecting communities of highly related features of the feature-read pairs,
thereby quantifying expression of RNA sequence reads, including communities of RNA sequence reads.

2. The computer-implemented method of claim 1, wherein the method further comprises outputting the quantification of RNA expression in an RNA expression matrix.

3. The computer-implemented method of claim 1 or claim 2, wherein the method further comprises assigning a biotype to a plurality of the detected communities.

4. The computer-implemented method of any one of claims 1-3, further comprising:
prior to (a), the RNA sequence reads are collected or have been collected from a library of detected RNA sequences (RNA-seq), wherein optionally the method further comprises trimming the collected RNA sequence reads to remove artificial sequences.

5. The computer-implemented method of any one of claims 1-4, wherein the hierarchical assignation of (a) comprises assigning RNA sequence reads that map to overlapping coordinates of the genome.

6. The computer-implemented method of any one of claims 1-5, wherein the hierarchical assignation of (a) comprises assigning RNA sequence reads overlapping a smalIRNA feature and a longRNA feature as smalIRNA, followed by assigning RNA sequence reads as long exonic RNA, followed by assigning remaining RNA sequence reads as long intronic RNA.

7. The computer-implemented method of any one of claims 1-6, wherein, prior to (a), the RNA sequence reads are aligned or have been aligned to a reference genome.

8. The computer-implemented method of any one of claims 1-7, wherein (b) further detects communities from a graph of feature-read pairs, wherein each vertex of the graph is a transcriptomic feature and edges connect two vertices for which multi-mapping reads exist, optionally wherein the edges between two vertices comprise pairs of edges.

9. The computer-implemented method of claim 8, wherein each vertex is weighted to have a size proportional to its number of aligned reads, wherein (b) accounts for vertex weights.

10. The computer-implemented method of claim 8 or claim 9, wherein each edge is weighted to have a thickness proportional to the fraction of multi-mappers shared by the vertices the edge connects, over total aligned reads, wherein (b) accounts for edge weights.

11. The computer-implemented method of any one of claims 1-10, wherein (b) comprises performing the Rosvall Map Equation to detect highly related communities.

12. The computer-implemented method of any one of claims 1-11, wherein the at least one cell is a single cell.

13. The computer-implemented method of any one of claims 1-11, wherein the at least one cell comprises multiple cells.

14. A computer-readable medium for performing the computer-implemented method of any one of the preceding claims.

15. A system, comprising a processor and instructions configured to cause the processor to perform the computer-implemented method of any one of claims 1-13.
